(19) Europäisches Patentamt
European Patent Office
Office européen des brevets

(11) Veröffentlichungsnummer: **0 258 493**
**B1**

(12) **EUROPÄISCHE PATENTSCHRIFT**

(45) Veröffentlichungstag der Patentschrift: **13.06.90**

(51) Int. Cl.⁵: **C09B 62/503,** D06P 1/384, C07C 315/04, C07C 317/02

(21) Anmeldenummer: **86117710.3**

(22) Anmeldetag: **19.12.86**

(54) **Wasserlösliche Triphendioxazin-Verbindungen und deren sulfonylgruppenhaltigen Vorprodukte, Verfahren zu deren Herstellung und Verwendung der Triphendioxazine als Farbstoffe.**

(30) Priorität: **19.08.86 DE 3628084**

(43) Veröffentlichungstag der Anmeldung: **09.03.88 Patentblatt 88/10**

(45) Bekanntmachung des Hinweises auf die Patenterteilung: **13.06.90 Patentblatt 90/24**

(84) Benannte Vertragsstaaten: **CH DE FR GB IT LI**

(56) Entgegenhaltungen: **DE-A- 3 439 755**

(73) Patentinhaber: **HOECHST AKTIENGESELLSCHAFT, Postfach 80 03 20, D-6230 Frankfurt am Main 80(DE)**

(72) Erfinder: **Springer, Hartmut, Dr., Am Erdbeerstein 27, D-6240 Königstein/Taunus(DE)**
Erfinder: **Helmling, Walter, Dr., Fichtestrasse 29, D-6238 Hofheim am Taunus(DE)**
Erfinder: **Schwaiger, Günther, Dr., Johannesallee 41, D-6230 Frankfurt am Main 80(DE)**

EP 0 258 493 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung liegt auf dem Gebiet der faserreaktiven Farbstoffe.

In den europäischen Patentanmeldungs-Veröffentlichungen Nrs. 0 141 996 A und 0 168 751 A sind einige faserreaktive Triphendioxazin-Farbstoffe mit Carbonamidgruppen beschrieben, die jedoch noch verbesserungswürdige Eigenschaften besitzen.

Es wurden nunmehr neue wasserlösliche Triphendioxazin-Ver-bindungen entsprechend der allgemeinen Formel (1)

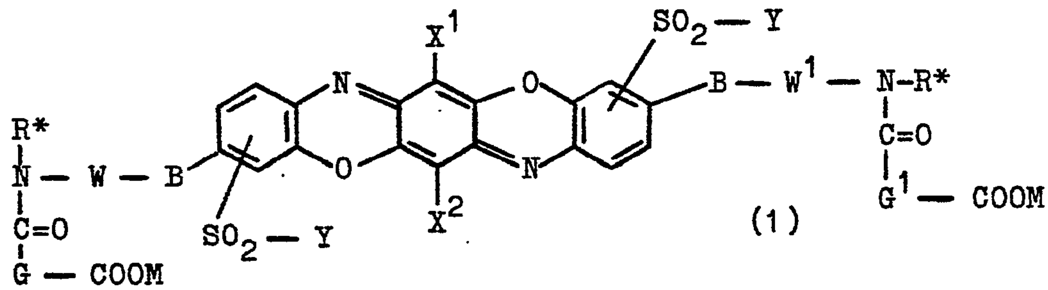

(1)

gefunden, die wertvolle faserreaktive Farbstoffeigenschaften besitzen.

In dieser Formel (1) bedeuten:

B ist ein Sauerstoff- oder Schwefelatom oder eine Aminogruppe der Formel -NH- oder -N(R')-, in welcher

R' eine Alkylgruppe von 1 bis 6 C-Atomen, bevorzugt von 1 bis 4 C-Atomen, wie die Methyl- oder Ethylgruppe, ist;

R* ist ein Wasserstoffatom oder eine gegebenenfalls substituierte Alkylgruppe von 1 bis 4 C-Atomen oder ein gegebenenfalls substituierter Arylrest, wie beispielsweise Methyl, Ethyl, Benzyl, Phenethyl, β-Sulfatoethyl, β-Sulfoethl, Phenyl, Sulfophenyl oder Sulfobenzyl;

W ist ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest, wobei die aliphatischen Reste durch Heterogruppen, bevorzugt 1 oder 2 Heterogruppen, unterbrochen sein können, die aus den Gruppen -0- , -S- , -$SO_2$- , -CO- , 1,4-Piperidino, -NH- und -N($R^0$) , worin $R^0$ eine der Bedeutungen von R' hat oder eine Alkanoylgruppe von 2 bis 5 C-Atomen, wie Acetylgruppe, ist, ausgewählt sind, und

$W^1$ hat eine der für W angegebenen Bedeutungen und ist mit W gleich oder von W verschieden, oder die Gruppierung -B-$W^1$ -N(R*)- und die Gruppierung -N(R*)-W-B-, zueinander gleich oder voneinander verschieden, stellt jede gemeinsam den bivalenten Rest eines fünf- oder sechsgliedrigen, zwei Stickstoffatome enthaltenden gesättigten Heterocyclus dar oder

die Gruppierung -B-$W^1$- und die Gruppierung -W-B-, zueinander gleich oder voneinander verschieden, stellt jede gemeinsam den bivalenten Rest eines fünf- oder sechsgliedrigen, zwei Stickstoffatome enthaltenden gesättigten Heterocyclus dar, der mit einem der beiden Stickstoffatome über eine Alkylengruppe von 2 bis 4 C-Atomen mit der Gruppierung -N(R*)-CO-$G^1$- bzw. -G-CO-N(R*)- verbunden ist;

G ist eine direkte Bindung oder eine geradkettige oder verzweigte Alkylengruppe von 1 bis 8 C-Atomen, bevorzugt von 2 bis 6 C-Atomen, oder ein aliphatisch-cycloaliphatischer Rest oder ein cycloaliphatischer Rest mit jeweils 5 bis 8 C-Atomen, bevorzugt 6 C-Atomen, im Cycloaliphaten;

$G^1$ hat eine der für G angegebenen Bedeutungen und ist mit G gleich oder von G verschieden;

M ist ein Wasserstoffatom oder ein Alkalimetal, wie Natrium, Kalium und Lithium, oder das Äquivalent eines Erdalkalimetalls, wie beispielsweise des Calciums, insbesondere jedoch ein Alkalimetall;

$X^1$ ist ein Wasserstoffatom oder ein Halogenatom, wie Chlor- oder Bromatom, eine Alkylgruppe von bis 1 bis 4 C-Atomen, eine Alkoxygruppe von 1 bis 4 C-Atomen, eine Aryloxygruppe oder ein gegebenenfalls substituierter Arylrest;

$X^2$ ist mit $X^1$ gleich oder von $X^1$ verschieden und hat eine der für $X^1$ angegebenen Bedeutungen;

Y ist die Vinylgruppe oder eine Ethylgruppe, die in β-Stellung einen durch ein Alkali eliminierbaren Substituenten enthält;

die Gruppe -$SO_2$-Y steht bevorzugt in ortho-Stellung zum Rest B gebunden.

Die einzelnen, auch zweifach erscheinenden Formelglieder, können zueinander gleiche oder voneinander verschiedene Bedeutungen besitzen; vorzugsweise besitzen sie zueinander gleiche Bedeutungen, weswegen die Verbindungen (1) bevorzugt symmetrisch aufgebaute Verbindungen sind.

Aliphatische Reste sind bevorzugt geradkettige oder verzweigte Alkylengruppen von 1 bis 6 C-Atomen. Substituierte Alkylgruppen sind beispielsweise solche, die durch 1 oder 2 Substituenten aus der Gruppe Chlor, Alkoxy, von 1 bis 4 C-Atomen, Benzoylamino, Sulfobenzoylamino, Alkanoylamino von 2 bis 5 C-Atomen, Hydroxy, Sulfato, Phosphato, Alkanoyloxy von 2 bis 5 C-Atomen, Sulfo, Carboxy oder

gegebenenfalls substituiertes Aryl substituiert sein können. Bevorzugte Substituenten sind hiervon die Carboxy- und Sulfogruppen sowie Sulfatogruppen.

Arylreste in den oben genannten oder nachstehend genannten Gruppen sind insbesondere die Phenyl- und Naphthylreste; sie können substituiert sein, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Nitro, Sulfamoyl und Carbamoyl (wobei Sulfamoyl und Carbamoyl durch gegebenenfalls substituiertes Alkyl von bis 4 C-Atomen und/oder gegebenenfalls substituiertes Aryl mono- oder disubstituiert sein können).

Bevorzugt ist W bzw. $W^1$ ein Alkylenrest von 2 bis 4 C-Atomen oder, falls er durch eine oder zwei Heterogruppen unterbrochen ist, ein Alkylenrest von 2 bis 6, insbesondere von 2 bis 4 C-Atomen, wobei im Alkylenrest die Heterogruppe bevorzugt ein Sauerstoffatom oder die Gruppe -NH- oder $-N(CH_3)-$ ist. Weiterhin sind W bzw. $W^1$ beispielsweise Cycloalkylengruppen von 5 oder 6 C-Atomen mit 1 bis 3 Methylgruppen als Substituenten oder zwei solche mit einem Alkylenrest von 1 bis 4 C-Atomen verbundene Cycloalkylengruppen oder ein Alkylenrest von 2 bis 6 C-Atomen, der durch eine solche Cycloalkylengruppe unterbrochen sein kann.

Reste $W^1$ sind beispielsweise der 1,2-Ethylen-, 1,3-Propylen-, 1,4-Butylen-, 1,5-Pentylen-, 1,6-Hexylen-, 2-Methyl-1,3-propylen-, 1,2-Dimethyl-1,2-ethylen-, 1,3-Dimethyl-1,3-propylen-, 2,2-Dimethyl-1,3-propylen- oder 1,2-, 1,4- und 1,3-Cyclohexylen-Rest oder ein bivalenter Rest der nachstehend angegebenen Formeln (a) bis (s), hiervon bevorzugt der 1,2-Ethylen-, 1,3-Propylen- und 1,4-Butylen-Rest und der Rest der Formel (a) :

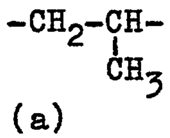
(a)

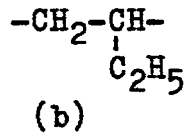
(b)

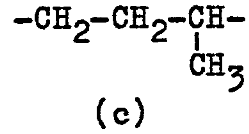
(c)

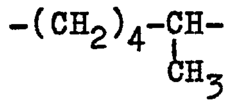
(d)

$-CH_2-CH_2-O-CH_2-CH_2-$ (e)

$-CH_2-CH_2-S-CH_2-CH_2-$ (f)

$-CH_2-CH_2-SO_2-CH_2-CH_2-$ (g)

$-(CH_2)_3-O-CH_2-CH_2-O-(CH_2)_3-$ (h)

$-CH_2-CH_2-NH-CH_2-CH_2-$ (j

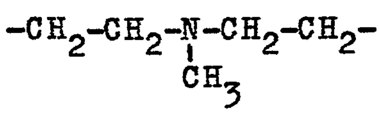
(k)

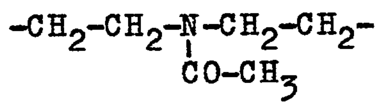
(m)

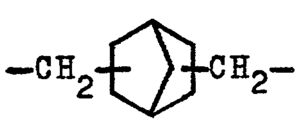
(n)

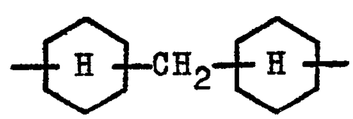
(p)

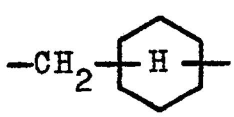
(r)

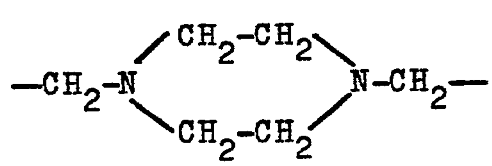
(s).

Die Reste W sind beispielweise die eben für $W^1$ genannten, jedoch "spiegelbildlich" angeordneten Gruppen.

Heterocyclische Gruppierungen, die sich gemeinsam aus den Formelresten -B-$W^1$ -N(R*)- bzw. -N(R*)-W-B- bilden, sind beispielsweise der bivalente 1,4-Piperidino-Rest. Gruppierungen mit heterocyclischen Resten, die sich gemeinsam aus dem Formelrest -B-$W^1$- bilden, sind beispielsweise der Rest der Formel (t)

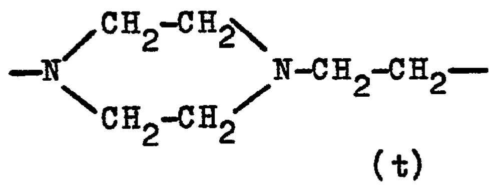

(t)

Gruppierungen mit heterocyclischen Resten, die sich gemeinsam aus dem Formelrest -W-B- bilden, sind beispielsweise ein Rest der Formel (u)

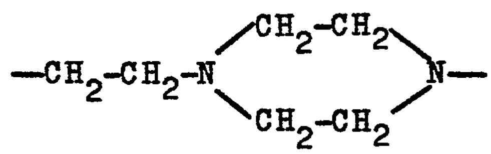

(u).

Bevorzugt bedeutet weiterhin B die Gruppe NH und R* ein Wasserstoffatom.

Aliphatische und cycloaliphatische Reste $G^1$ sind beispielsweise der 1,2-Ethylen-, 1,3-Propylen-, 1,4-Butylen-, 1,5-Pentylen-, 2-Methyl-1,3-propylen-, 1,2-Dimethyl-1,2-ethylen-, 2-Ethyl-1,3-propylen-, 2,2-Dimethyl-1,3-propylen-, 2-(n- oder iso-Propyl)-1,3-propylen-, 1,2,2-Trimethyl-1,2-ethylen-, 1,2-Diethyl-1,2-ethylen-, 1,1,2,2-Tetramethyl-1,2-ethylen-, 2-Methyl-2-ethyl-1,3-propylen-, 1,1,3,3-Tetramethyl-1,3-propylen-, 1,2-Cyclohexylen- oder 1,4-Cyclohexylen-Rest oder ein bivalenter Rest der nachstehend angegebenen Formeln (a*) bis (h*), hiervon bevorzugt der 1,2-Ethylen-, 1,3-Propylen-, 2-Methyl-1,3-propylen-, 2,2-Dimethyl-1-1,3-propylen- und 1,2-Cyclohexylenrest und der Rest der Formel (a*):

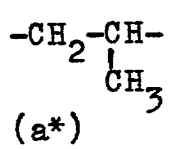
(a*)

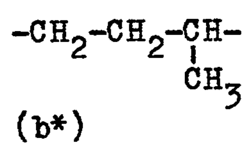
(b*)

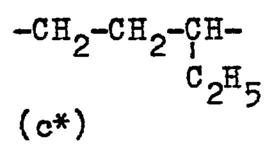
(c*)

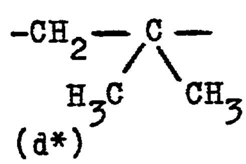
(d*)

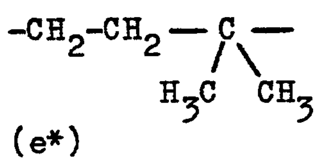
(e*)

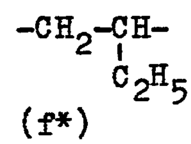
(f*)

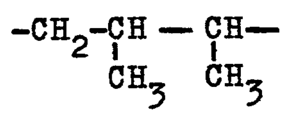
(g*)

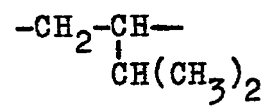
(h*)

Die Reste sind beispielsweise die eben für $G^1$ genannten, jedoch "spiegelbildlich" angeordneten Gruppen.

Bevorzugt sind die Formelreste $X^1$ und $X^2$, zueinander gleich oder voneinander verschieden, bevorzugt zueinander gleich, jedes ein Wasserstoffatom oder insbesondere ein Halogenatom, wie ein Bromatom und insbesondere ein Chloratom.

Im vorstehenden und nachfolgenden sind Sulfogruppen Gruppen entsprechend der allgemeinen Formel $-SO_3M$, Carboxygruppen Gruppen entsprechend der allgemeinen Formel -COOM, Sulfatogruppen Gruppen entsprechend der allgemeinen Formel $-OSO_3M$, Thiosulfatogruppen Gruppen entsprechend der allgemeinen Formel $-S-SO_3M$ und Phosphatogruppen Gruppen entsprechend der allgemeinen Formel $-OPO_3M_2$, wobei M jeweils die oben angegebene Bedeutung besitzt.

Die neuen Dioxazinverbndungen können sowohl in saurer Form als auch in Form ihrer Salze vorliegen. Bevorzugt sind sie in Form der Alkalisalze und finden auch bevorzugt in Form dieser Salze Verwendung zum Färben (im allgemeinen Sinne und einschließlich des Bedruckens verstanden) von hydroxy- und/oder carbonamidgruppenhaltigen Materialien, insbesondere Fasermaterialien.

Bevorzugte erfindungsgemäße Triphendioxazin-Verbindungen sind solche der allgemeinen Formel (1a)

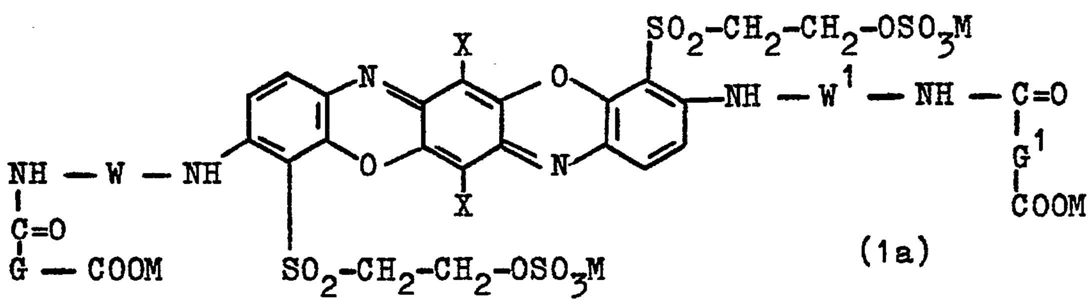

(1a)

in welcher
W und $W^1$ beide eine Alkylengruppe von 2 bis 6 C-Atomen bedeuten,
X jedes für ein Bromatom oder insbesondere bevorzugt ein Chloratom steht,
M die oben genannte Bedeutung besitzt und bevorzugt ein Alkalimetall ist und
G und $G^1$ beide eine Alkylengruppe von 2 bis 6 C-Atomen bedeuten.

Bevorzugt sind insbesondere von den Verbindungen der allgemeinen Formel (1a) diejenigen, in welchen beide X für ein Chloratom stehen, des weiteren solche, in welchen W und $W^1$ beide den 1,2-Ethylenrest oder 1,3-Propylenrest bedeuten und G und $G^1$ beide den 1,2-Ethylenrest bedeuten.

Die vorliegende Erfindung betrifft weiterhin Verfahren zur Herstellung der obengenannten und definierten Verbindungen der allgemeinen Formel (1). Diese sind dadurch gekennzeichnet, daß man eine Verbindung der allgemeinen Formel (2)

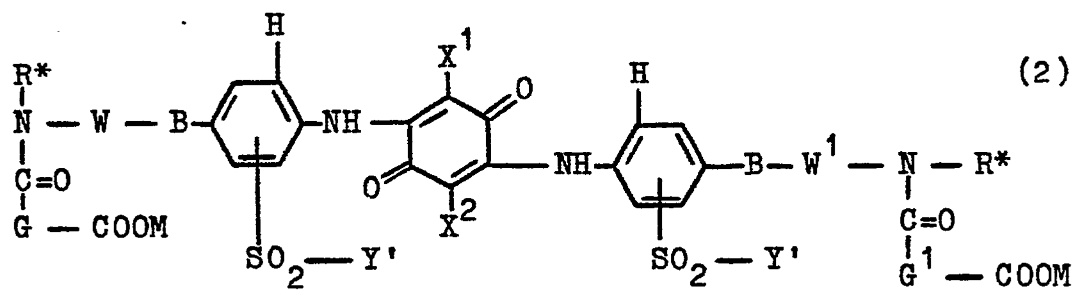

(2)

(in welcher Y' die Vinylgruppe, die β-Hydroxyethyl-Gruppe oder eine Ethylgruppe, die in β-Stellung einen durch ein Alkali eliminierbaren Substituenten, wie einen der für Y genannten, enthält, bevorzugt die β-Hydroxyethyl-Gruppe ist und B, G, $G^1$, R*, W, $W^1$, $X^1$ und $X^2$ die obengenannten Bedeutungen haben, wobei substituierte Alkylgruppen in diesen Resten auch hydroxysubstituierte Alkylgruppen sein können, die Gruppen $-SO_2-Y'$ bevorzugt in ortho-Stellung zur Gruppe B gebunden sind und die Benzolkerne in einer der ortho-Stellungen zur angegebenen Aminogruppe -NH- nicht substituiert sein dürfen) in saurem Medium und vorzugsweise in Gegenwart eines Oxidationsmittels zum Triphendioxazin cyclisiert. Die Umsetzung erfolgt in an und für sich bekannter Verfahrensweise, so beispielsweise in Schwefelsäure oder Schwefeltrioxid enthaltender Schwefelsäure als Reaktionsmedium, wobei als Oxidationsmittel Schwefeltrioxid, Ammonium- oder Alkalipersulfate, Jod oder anorganische Jodverbindungen in Gegenwart von Oleum, Natriumborat, vorzugsweise jedoch Natrium- oder Kaliumperoxodisulfat (entsprechend den Formeln $Na_2S_2O_8$ bzw. $K_2S_2O_8$), verwendet werden. Solche Verfahrensweisen sind bspw. aus der britischen Patentschrift Nr. 1 589 915 und den europäischen Patentanmeldungs-Veröffentlichungen Nrs. 0 141 359A und 0 168 751 A bekannt.

Vorzugsweise führt man die Cyclisierung in konzentrierter Schwefelsäure, wie 96- bis bevorzugt 100 %iger Schwefelsäure und insbesondere in Schwefeltrioxid enthaltender Schwefelsäure (Oleum), wie bis zu 50 gew.-%igem Oleum, durch. Die Reaktionstemperatur wird zwischen 0 und 60°C gewählt. Das als Reaktionsmedium und Agenz verwendete Oleum besitzt in der Regel einen Gehalt von 5 bis 40 Gew.-%, bevorzugt 10 bis 20 Gew.-% an Schwefeltrioxid. Bei Zusatzvon Peroxodisulfat als Oxidationsmittel führt man die Cyclisierung zwischen 0 und 40°C, bevorzugt zwischen 15 und 25°C durch. Bei Verwendung von Oleum/Peroxodisulfat soll die Reaktionstemperatur 30°C nicht überschreiten. Bevorzugt ist 10 bis 20%iges Oleum unter Verwendung einer zur Verbindung (2) doppelt molaren Menge Peroxodisulfat. Bei

Jod als Oxidationsmittel wird dieses in katalytischen Mengen in 10 bis 50%igem Oleum eingesetzt; hier liegt die Reaktionstemperatur in der Regel zwischen 0 und 40°C.

Gleichzeitig mit der Cyclisierung oder erst nach der Cyclisierungsreaktion können gegebenenfalls vorhandene Hydroxyalkylgruppen, wie beispielsweise die β-Hydroxyethyl-Gruppe des Formelrestes Y', mittels eines Sulfatisierungs- oder Phosphatierungsmittels, wie 96-100%iger Schwefelsäure oder Schwefeltrioxid enthaltender Schwefelsäure bzw. Polyphosphorsäure, in die entsrechenden β-Sulfatoalkyl- bzw. β-Phosphatoalkyl-Gruppen verestert werden. Wird also der Ringschluß in Schwefelsäure oder Oleum als Reaktionsmedium ausgeführt, werden Hydroxygruppen, die an einem Alkylrest des Moleküls gebnden sind, wie beispielsweise die oben bereits erwähnten β-Hydroxyethyl-Gruppen des Formelrestes Y' oder Hydroxyalkyl-Gruppen der Formelreste R*, W und $W^1$, in die entsprechenden Sulfatoalkylgruppen übergeführt.

Bei Cyclisierungstemperaturen von oberhalb 15°C, insbesondere oberhalb 25°C, lassen sich mittels 100 %iger Schwefelsäure oder Oleum erfindungsgemäß auch Sulfogruppen in die aromatischen Ringe der Arylreste von R* , $X^1$ und $X^2$ einführen. Die Sulfierungsreaktion kann aber auch nach der Cyclisierung, gegebenenfalls in einem gesonderten Reaktionsschritt beim isolierten Cyclisierungsprodukt (Triphendioxazin), erfolgen. Hier wird die Sulfierungsreaktion in der Regel zwischen 20 und 80°C, bevorzugt zwischen 30 und 70°C, geführt.

Verbindungen der Formel (1) mit Y gleich einer β-Sulfatoethyl-Gruppe können anschließend gemäß bekannten Verfahrensweisen in andere erfindungsgemäße Verbindungen der Formel (1) übergeführt werden, in welcher die für Vinylgruppen oder Ethylgruppen mit einem anderen in β-Stellung befindlichen alkalisch eliminierbaren Substituenten stehen.

Die Ausgangsverbindungen der allgemeinen Formel (2) können in ebenfalls erfindungsgemäßer Weise durch Umsetzung einer Verbindung der allgemeinen Formel (3)

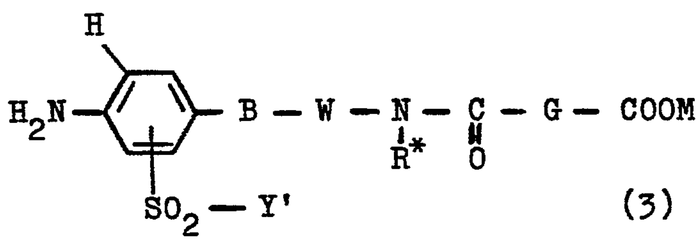

(3)

(worin W auch für $W^1$ und G auch für $G^1$ steht und Y' die obengenannte Bedeutung besitzt und bevorzugt die β-Hydroxyethyl-Gruppe ist und R* und B die obengenannten Bedeutungen haben, wobei substituierte Alkylgruppen in die sen Resten auch hydroxysubstituierte Alkylgruppen sein können und Arylreste auch von Sulfogruppen frei sein können und die Gruppen $-SO_2-Y'$ bevorzugt in ortho-Stellung zur Gruppe B gebunden sind) mit einer 1,4-Benzochinon-Verbindung der allgemeinen Formel (4)

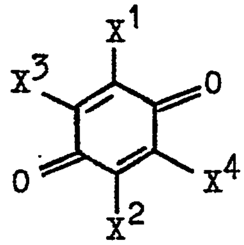

(4)

in welcher $X^1$ und $X^2$ die obengenannten Bedeutungen haben und $X^3$ und $X^4$ zueinander gleich oder voneinander verschieden sind und jedes für ein Wasserstoffatom, eine Alkoxygruppe von 1 bis 4 C-Atomen, wie insbesondere die Methoxygruppe, oder für eine Phenoxygruppe steht oder bevorzugt ein Halogenatom, wie Fluoratom, besonders Bromatom und insbesondere Chloratom, ist, wobei $X^3$ und $X^4$ auch eine zu $X^1$ und $X^2$ gleiche Bedeutung haben können, hergestellt werden.

Die Umsetzung einer Verbindung der allgemeinen Formel (3) oder zweier verschiedener Aminoverbindungen der allgemeinen Formel (3), jeweils in zusammen 2-fach äquivalenter Menge, mit einem Äquivalent einer Verbindung der allgemeinen Formel (4) zur Verbindung der allgemeinen Formel (2) erfolgt analog bekannten Verfahrensweisen, die beispielsweise in den europäischen Patentanmeldungs-Veröffentlichungen Nrs. 0 141 996A und 0 168 751 A erwähnt und beschrieben sind. Beispielsweise kann die Umsetzung in wäßrigen Medium oder in wäßrig-organischem Medium oder in rein organischem Medium erfolgen, wobei die organischen Lösemittel polare aprotische und protische Lösungsmittel darstellen, wie beispielsweise niedere Alkanole, wie Methanol und Ethanol, und halogenierte Benzole, wie o-Dichlorbenzol. Bevorzugt wird das Chinon (4) jedoch in einem mehr oder weniger stärkeren Überschuß eingesetzt,

der in der Regel 5 bis 20 % beträgt. Die Umsetzung der Amine (3) mit den Chinonen (4) kann bei einer Temperatur zwischen 20 und 100°C, vorzugsweise zwischen 50 und 70°C, in Gegenwart eines säurebindenden Mittels, wie beispielsweise eines Alkali- oder Erdalkalicarbonats oder -acetats, so beispielsweise Natriumacetat, Natriumcarbonat oder Natiumbicarbonat, oder eines Alkali- oder Erdalkalihydroxids, wie Natriumhydroxid, oder eines Oxids eines Erdalkalimetalls, wie beispielsweise Magnesiumoxid, durchgeführt werden. Sofern in einem wäßrigen oder wäßrig-organischen Medium gearbeitet wird, wird ein pH-Bereich zwischen 4 und 7, vorzugsweise zwischen 5,5 und 6,5 eingestellt.

Die Anilin-Ausgangsverbindungen der allgemeinen Formel (3) sind bisher noch nicht bekannt. Die Erfindung betrifft somit auch diese Verbindungen, Verfahren zu deren Herstellung und ihre Verwendung zur Synthese von Farbstoffen. Sie lassen sich analog bekannten Verfahrensweisen der Umsetzung von Aminoverbindungen mit Carbonsäureanhydriden herstellen, so beispielsweise erfindungsgemäß durch Umsetzung einer Aminoverbindung der allgemeinen Formel (5)

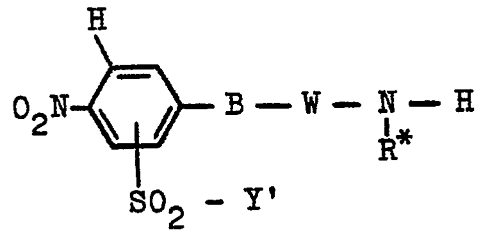
(5)

in welcher Y', B, W und R* die oben genannten Bedeutungen haben, mit einem Anhydrid der allgemeinen Formel (6) oder (7)

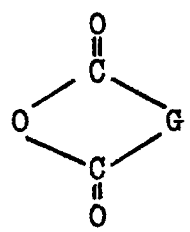
(6)

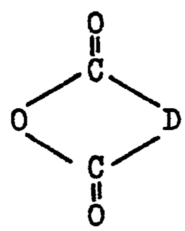
(7)

in welchen G die obengenannte Bedeutung besitzt und D einen bivalenten, geradkettigen oder verzweigten ungesättigten, eine oder zwei Doppelbindungen enthaltenden Rest von 2 bis 8 C-Atomen bedeutet, und anschließende Reduktion der Nitrogruppe und einer gegebenenfalls vorhandenen olefinischen Bindung.

Ungesättigte aliphatische Reste D sind beispielsweise die Reste der Formeln -CH=CH- , $-CH_2-CH=CH-$ , $-CH=C(CH_3)-$ , $-CH_2-C(=CH_2)-$ , $-CH=CH-CH(CH_3)-$ , $-CH_2-C(CH_3)=CH-$ , $-CH=(C_2H_5)-$ ,

-CH=CH- , $-CH_2-CH=CH-$ , $-CH=C(CH_3)-$ ,

$-CH_2-C(=CH_2)-$ , $-CH=CH-CH(CH_3)-$ , $-CH_2-C(CH_3)=CH-$ ,

$-CH=(C_2H_5)-$ , 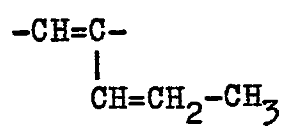 ,

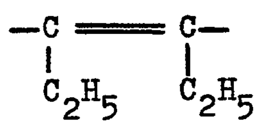 und 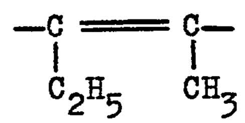 .

Bevorzugt bedeutet D den Vinylidenrest.

Die Reaktion der Verbindungen der allgemeinen Formel (5) mit den Verbindungen (6) oder (7) erfolgt gemäß den in der Literatur für diese Reaktionsart beschriebenen Verfahrensweisen, wie beispielsweise in wäßrigem Medium oder auch in einem für diese Reaktionsart gängigen organischen Medium, wie in einem Alkanol von 1 bis 6 C-Atomen, wie insbesondere Methanol oder Ethanol, Aceton, Methylethylketon, Dioxan, N,N-Dimethylformamid, N-Methyl-pyrrolid-2-on oder Toluol oder einem Gemisch von Wasser

und einem wassermischbaren organischen Lösemittel, bei einer Temperatur zwischen 0 und 100°C und, sofern in wäßrigem oder wäßrig-organischem Medium gearbeitet wird, bei einem pH-Wert zwischen 2 und 10.

Carbonsäureanhydride der allgemeinen Formel (6) sind beispielsweise Bernsteinsäureanhydrid, Glutarsäureanhydrid, Adipinsäureanhydrid, 3-Methyl-bernsteinsäureanhydrid, 3-Ethyl-bernsteinsäureanhydrid, 3,4-Dimethyl-bernstein säureanhydrid, 3,3-Dimethyl-bernsteinsäureanhydrid, 4-Methylglutarsäureanhydrid, 3,3-Dimethyl-glutarsäureanhydrid, 3,4-Dimethyl-glutarsäureanhydrid, 4,4-Dimethylglutarsäureanhydrid, 3-Isopropyl-bernsteinsäureanhydrid, 3,4-Diethyl-bernsteinsäureanhydrid, 3,3,4,4-Tetramethylbernsteinsäureanhydrid, 3,3,5,5-Tetramethyl-glutarsäureanhydrid, 4-Methyl-4-ethyl-glutarsäureanhydrid und Cyclohexan-1,2-dicarbonsäureanhydrid.

Ungesättigte Carbonsäureanhydride der allgemeinen Formel (7) sind beispielsweise Maleinsäureanhydrid, 3-Methylmaleinsäureanhydrid oder das Anhydrid der Prop-2-en-1,3-dicarbonsäure, der 2-Methyl-prop-2-en-1,3-dicarbonsäure oder der Prop-2-en-1,2-dicarbonsäure.

Die aus den Reaktionen der Verbindungen (5) mit den Verbindungen (6) bzw. (7) erhaltenen, in der Literatur noch nicht beschriebenen erfindungsgemäßen Nitroverbindungen entsprechend einer allgemeinen Formel (8) oder (9)

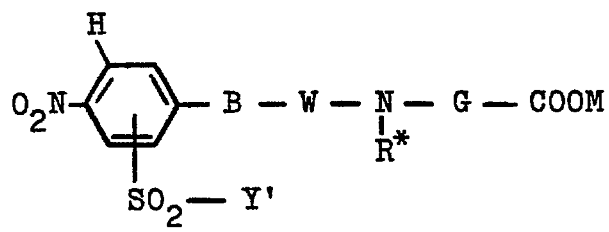

(8)

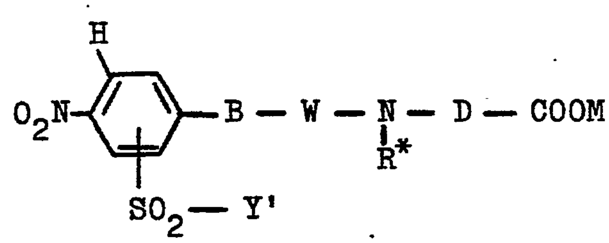

(9)

in welchen Y', B, W, D, G, R* und M die oben genannten Bedeutungen haben, können sodann nach an und für sich üblichen Methoden der Reduktion von aromatischen Nitrogruppen mittels Wasserstoff an einem metallischen Katalysator, wie einem Palladium, Platin- oder Raney-Nickelkatalysator, unter Druck im Autoklaven oder mittels der Reduktion nach Béchamp bei Verwendung von Eisenspänen zur Verbindung der allgemeinen Formel (3) überführt werden. Hierbei werden in den Verbindungen (10) auch die ungesättigten Bindungen von D unter Bildung der Verbindung (3) hydriert.

Die Ausgangsverbindungen entsprechend der allgemeinen Formel (5) sind bekannt oder können analog hierzu in an und für sich üblicher Weise hergestellt werden, wie beispielsweise nach den Angaben der deutschen Offenlegungsschrift Nr. 3 502 991.

Ausgangsverbindungen der allgemeinen Formel (3) sind beispielsweise 3-(β-Hydroxyethylsulfonyl)-4-N-[β-(bernsteinsäure-monoamido)-ethyl]-amino-anilin, 3-(β-Hydroxyethylsulfonyl)-4-N-[γ-(bernsteinsäure-monoamido)-n-propyl]-amino-anilin, 3-(β-Hydroxyethylsulfonyl)-4-N-[β-(bernsteinsäure-monoamido)-n-propyl]-amino-anilin, 3-(β-Hydroxyethylsulfonyl)-4-N-[β-(1'-carboxy-cyclohexan-2'-carbonamido)-n-propyl]-amino-anilin, 3-(β-Hydroxyethylsulfonyl)-4-N-[β-(1'-carboxy-cyclohexan-2'-carbonamido)-ethyl]-amino-anilin, 3-(β-Hydroxyethylsulfonyl)-4-N-{β-[β'-(bernsteinsäure-monoamido)-ethoxy]-ethyl)-amino-anilin, 3-(β-Hydroxyethylsulfonyl)-4-N-[β-(2'-carboxy-n-propan-1'-carbonamido)-ethyl]-amino-anilin, 3-(β-Hydroxyethylsulfonyl)-4-N-{β-[β'-(bernsteinsäure-monoamido)-ethylsulfonyl]-ethyl}-amino-anilin, 3-(β-Hydroxyethylsulfonyl)-4-N-[β-(glutarsäure-monoamido)-ethyl]-amino-anilin, 3-(β-Hydroxyethylsulfonyl)-4-N-[γ-(glutarsäure-monoamido)-n-propyl]-amino-anilin, 3-(β-Hydroxyethylsulfonyl)-4-N-[β-(3'-carboxy-2'-methylpropan-1'-carbonamido)-ethyl]-amino-anilin, 3-(β-Hydroxyethylsulfonyl)-4-N-[β-(2'-carboxy-2'-methylpropan-1'-carbonamido)-ethyl]-amino-anilin, 3-(β-Hydroxyethylsulfonyl)-4-N-[γ-(2'-carboxy-2'-methyl-propan-1'-carbonamido)-n-propyl]-amino-anilin, 3-(β-Hydroxyethyl sulfonyl)-4-N-[β-(3'-carboxy-2',2'-dimethyl-propan-1'-carbonamido)-ethyl]-amino-anilin, 3-(β-Hydroxyethylsulfonyl)-4-N-[γ-(1'-carboxy-cyclohexan-2'-carbonamido)-propyl]-amino-anilin und 3-(β-Hydroxyethylsulfonyl)-4-N-{β-[β'-(bernsteinsäure-monoamido)-N'-methyl-N'-ethyl]-ethyl}-aminoanilin.

Die als Ausgangsverbindungen dienenden Benzochinone der allgmeinen Formel (4) sind beispielsweise 2-Methyl-3,5,6-tribrom-1,4-benzochinon, 2-Methoxy-3,5,6-trichlor-1,4-benzochinon, 2,3,5,6-Tetra-

methoxy-1,4-benzochinon, 2,3,5,6-Tetraphenoxy-1,4-benzochinon, 2-Methyl-3,6-dichlor-1,4-benzochinon, 2,5-Dichlor-1,4-benzochinon und bevorzugt 2,3,5,6-Tetrabrom-1,4-benzochinon und insbesondere 2,3,5,6-Tetrachlor-1,4-benzochinon (Chloranil).

Die Abscheidung und Isolierung der erfindungsgemäß hergestellten Verbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Puffersubstanz zugefügt werden kann.

Die erfindungsgemäßen Verbindungen der allgemeinen Formel (1) - im nachfolgenden als Verbindungen (1) bezeichnet - haben faserreaktive Eigenschaften und besitzen wertvolle Farbstoffeigenschaften. Sie können deshalb zum Färben (einschließlich Bedrucken) von natürlichen, regenerierten oder synthetischen hydroxygruppenhaltigen und/oder carbonamidgruppenhaltigen Materialien, beispielsweise in Form von Flächengebilden, wie Papier und Leder, oder in der Masse, von Polyamid oder Polyurethan, insbesondere aber von solchen Materialien in Faserform, wie Cellulosefasermate rialien, Seide, Wolle und synthetischen Polyamid- und Polyurethanfasern, verwendet werden. Auch können die bei der Synthese der Verbindungen (1) anfallenden Lösungen, gegebenenfalls nach Zustaz einer Puffersubstanz, gegebenenfalls auch nach Konzentrierung, direkt als Flüssigpräparation der färberischen Verwendung zugeführt werden.

Die Verbindungen (1) lassen sich, gemäß der erfindungsgemäßen Anwendung, auf den genannten Substraten, insbesondere auf den genannten Fasermaterialien, nach den für wasserlösliche, insbesondere faserreaktive, Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solche Färbe- und Fixierweisen sind in der Literatur zahlreich beschrieben, wie beispielsweise auch in der europäischen Patentanmeldungs-Veröffentlichung Nr. 0 168 751 A.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen der Applikation und Fixierung von faserreaktiven Farbstoffen vorgehen.

Die mit den Verbindungen (1) hergestellten Färbungen und Drucke zeichnen sich durch reine, vorwiegend blaue Farbtöne aus. Insbesondere die Färbungen und Drucke auf Cellulosefasermaterialien besitzen sehr hohe Farbstärken und ebenso sehr gute Lichtechtheiten, einschließlich guter Naßlicht- und Schweißlichtechtheiten, ebenso gute Hypochloritbleich- und Chlorbadewasserechtheiten, weiterhin vorzügliche Naßechtheiten, wie beispielsweise gute Waschechtheiten bei 60 bis 95°C, auch in Gegenwart von Perboraten, saure und alkalische Walk-, Überfärbe- und Schweißechtheiten, Alkali-, Säure-, Wasser- und Seewasserechtheiten, desweiteren eine gute Plissierechtheit, Bügelechtheit und Reibechtheit. Ebenso besitzen sie eine gute Naßliegeechtheit und eine sehr gute Säurelagerbeständigkeit ("acid fading") beim Lagern von feuchten, noch Essigsäure enthaltendem, gefärbtem Material. Desweiteren sind die Färbungen gegen die üblichen Kunstharzappreturen stabil. Ein Teil der Verbindungen (1) sind in der Reinheit des Farbtones und in wichtigen Echtheitseigenschaften mit faserreaktiven Anthrachinonfarbstoffen vergleichbar.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Natrium- oder Kaliumsalze hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, wie Natrium- oder Kaliumsalze, in die Synthese eingesetzt werden.

Die Absorptionsmaxima ($\lambda_{max}$-Werte) wurden in wäßriger Lösung bestimmt.

Die $^1$H-NMR-Messungen erfolgten in $d_6$-Dimethylsulfoxid mit Tetramethylsilan als innerem Standard, sofern nicht anders angegeben.

Beispiel 1

a) Zu einer 60°C warmen Suspension von 14,4 Teilen 4-(β-Aminoethyl-amino)-3-(β-hydroxyethylsulfonyl)-nitrobenzol in 100 Teilen Wasser wird innerhalb von 15 Minuten langsam und stetig eine Lösung von 5,4 Teilen Maleinsäureanhydrid in 15 Teilen Aceton zugegeben. Danach wird bei 60°C noch 20 Minuten nachgerührt, der Ansatz sodann erkalten lassen, das ausgefallene Produkt abgesaugt und unter reduziertem Druck bei 60°C getrocknet.

Es besitzt folgende Analysendaten:

| | | | |
|---|---|---|---|
| Schmelzpunkt: 175°C | | | |
| Elementaranalyse (berechnet auf $C_{14}H_{17}N_3O_8S$ und MG = 387,3): | | | |
| berechnet: | C 43,4%, | H 4,4%, | N 10,8%, |
| gefunden: | C 43,4%, | H 4,4%, | N 10,8%; |

$^1$H-NMR-Spektroskopie:
δ = 3,41 ppm (m,2H); 3,49 ppm (m,2H); 3,53 ppm (t,2H); 3,71 ppm (t,2H); 4,83 ppm (s, OH); 6,21 ppm (d,1H); 6,35 ppm (d,1H); 7,08 ppm (d,1H); 7,30 ppm (t,NH); 8,24 ppm (dd,1H); 8,40 ppm (d,1H); 8,99 ppm (t,NH); 14,15 ppm (s,OH).

Die Verbindung besitzt die folgende Konstitution:

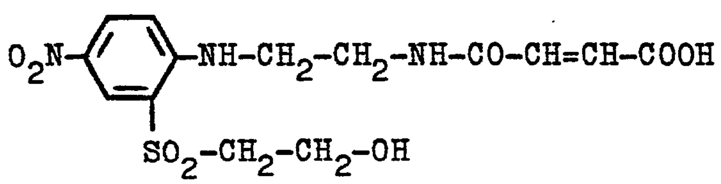

b) Eine 40°C warme Suspension von 60 Teilen 4-(β-Aminoethyl-amino)-3-(β-hydroxyethylsulfonyl)-nitrobenzol in 200 Teilen Wasser wird innerhalb von 15 Minuten langsam und stetig mit einer Lösung von 22 Teilen Bernsteinsäureanhydrid in 40 Teilen Aceton versetzt. Der Ansatz wird bei 40°C noch 30 Minuten nachgerührt und sodann abkühlen lassen; das ausgefallene Produkt wird abgesaugt und getrocknet.

Es bisitzt folgende Analysendaten:

| | | | |
|---|---|---|---|
| Schmelzpunkt: 143–145°C | | | |
| Elementaranalyse (berechnet auf $C_{14}H_{19}N_3O_8S$ und MG = 389,4): | | | |
| berechnet: | C 43,1% | H 4,9%, | N 10,8%, |
| gefunden: | C 43,1%, | H 5,0%, | N 10,5%; |

$^1$H-NMR-Spektroskopie:
δ = 2,32 ppm (t,2H); 2,42 ppm (t,2H); 3,28 ppm (m,2H); 3,43 ppm (m,2H); 3,50 ppm (t,2H); 3,72 ppm (t,2H); 7,08 ppm (d,1H); 7,28 ppm (t,NH); 8,09 ppm (t,NH); 8,27 ppm (dd,1H); 8,40 ppm (d,1H);
die Protonen von COOH und OH waren nicht sichtbar.

Die Verbindung besitzt die folgende Konstitution:

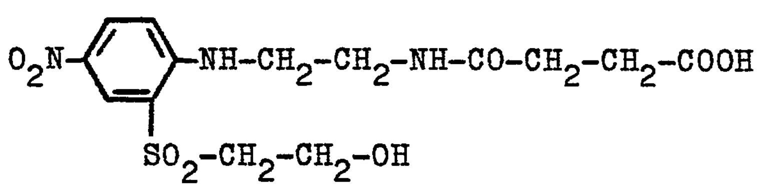

c) 39 Teile der Nitroverbindung von a) oder von b) werden in 200 Volumenteilen Methanol gelöst und in einem Autoklaven bei einer Temperatur von bis zu 80°C und einem Druck von 50 bar in Gegenwart eines Palladiumkatalysators hydriert. Die Hydrierdauer beträgt etwa 4 Stunden. Der Katalysator wird sodann abfiltriert und das Filtrat durch Abdestillation des Lösemittels eingeengt und von diesem befreit. Die gewünschte Verbindung der Formel

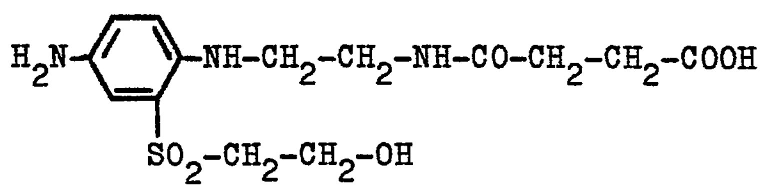

wird in Form eines Öles erhalten.

Die Verbindung besitzt folgende ¹H-NMR-spektroskopische Daten:
δ = 2,32 ppm (t,2H); 2,40 ppm (t,2H); 3,81 ppm (m,2H); 3,25 ppm (m,2H); 3,35 ppm (t,2H); 3,67 ppm (t,2H); 5,42 ppm (s,NH); 6,72 ppm (d,1H); 6,85 ppm (dd,1H); 6,95 ppm (d,1H); 8,03 ppm (s,NH);
die Protonen von COOH, $NH_2$ und OH waren nicht sichtbar.

Beispiel 2

a) Zur Synthese des erfindungsgemäßen Vorproduktes 3-(β-Hydroxyethylsulfonyl)-4-N-[γ-(maleinsäure-monoamido)-n-propyl-amino]-nitrobenzol verfährt man gemäß der Verfahrensweise des Beispieles 1a), setzt jedoch an Stelle von 4-(β-Aminoethyl-amino)-3-(β-hydroxyethylsulfonyl)-nitrobenzol die äquivalente Menge an 4-(γ-Amino-n-propyl-amino)-3-(β-hydroxyethylsulfonyl)-nitrobenzol ein.
Die erhaltene Verbindung besitzt folgende Analysendaten:
Schmelzpunkt: 154-156°C ;
¹H-NMR-Spektroskopie:
δ = 1,78 ppm (m,2H); 3,26 ppm (m,2H); 3,42 ppm (m,2H); 3,5 ppm (t,2H); 3,72 ppm (t,2H); 4,91 ppm (s,OH); 6,21 ppm (d,1H); 6,40 ppm (d,1H); 7,02 ppm (d,1H); 7,28 ppm (t,NH); 8,23 pm (dd,1H); 8,41 ppm (d,1H); 9,00 ppm (t,NH);
das Proton der COOH-Gruppe war nicht sichtbar.
Die Verbindung besitzt die folgende Konstitution:

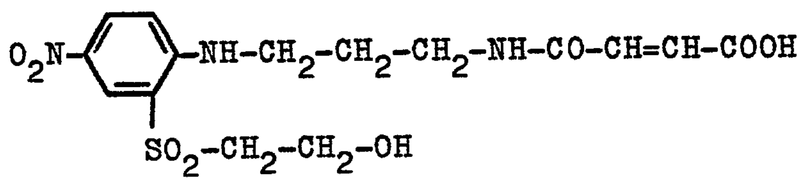

b) Das zu a) analoge erfindungsgemäße, im aliphatischen Carbonsäureanteil gesättigte Vorprodukt 3-(β-Hydroxy ethylsulfonyl)-4-N-[γ-(bernsteinsäure-monoamido)-n-propyl-amino]-nitrobenzol kann analog der Verfahrensweise des Beispieles 1b) hergestellt werden, wenn man anstelle der dortigen Ausgangsverbindung 4-(β-Aminoethyl-amino)-3-(β-hydroxyethylsulfonyl)-nitrobenzol die äquivalente Menge an 4-(γ-Amino-n-propylamino)-nitrobenzol einsetzt.
Die Verbindung besitzt folgende Analysendaten:
Schmelzpunkt: 412-144°C ;
¹H-NMR-Spektroskopie:
δ = 1,69 ppm (m,2H); 2,32 ppm (t,2H); 2,42 ppm (t,2H); 3,18 ppm (m,2H); 3,37 ppm (m,2H); 3,52 ppm (t,2H); 3,62 ppm (t,2H); 6,98 ppm (d,1H); 7,25 ppm (t,NH); 7,82 ppm (t,NH); 8,22 ppm (dd,1H); 8,39 ppm (d,1H);
die Protonen von COOH und OH waren nicht sichtbar.
Die Verbindung besitzt die folgende Konstitution:

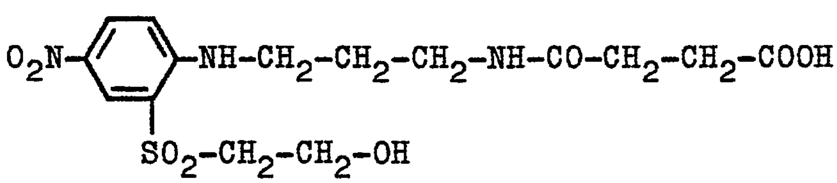

c) Die unter a) und ebenso die unter b) beschriebene Nitroverbindung können gemäß den Angaben des Beispieles 1c) zur Aminoverbindung der Formel

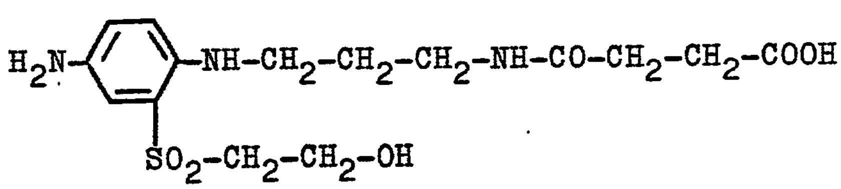

reduziert werden. Dieses Anilinderivat wird in Form eines Öles erhalten.
Es besitzt die folgenden ¹H-NMR-spektroskopische Daten:
δ = 1,62 ppm (m,2H); 2,25 ppm (t,2H); 2,40 ppm (t,2H); 3,03 ppm (m,2H); 3,06 ppm (m,2H); 3,34 ppm (t,2H); 3,63 ppm (t,2H); 5,32 ppm (s,NH); 6,66 ppm (d,1H); 6,82 ppm (dd,1H); 6,92 ppm (d,1H); 7,89 ppm (t,NH);
weitere Protonen waren nicht sichtbar.

Beispiel 3

Zur Herstellung einer erfindungsgemäßen Triphendioxazin-Verbindung geht man von einer wäßrigen Lösung mit einem pH-Wert von 6,0 von 54 Teilen der in Beispiel 1c) beschriebenen Anilinverbindung in 500 Teilen Wasser aus. Man erwärmt diese Lösung auf 60°C und setzt bei dieser Temperatur unter Einhaltung eines pH-Wertes zwischen 6,0 und 6,5 portionsweise insgesamt 19 Teile Chloranil hinzu. Der Ansatz wird noch 3 Stunden nachgerührt, das ausgefallene Produkt abgesaugt, mit etwas Wasser gewaschen und unter reduziertem Druck bei 80°C getrocknet.

Das erhaltene chinoide Kondensationsprodukt besitzt folgende $^1$H-NMR-spektroskopische Daten: δ = 2,32 ppm (t,4H); 2,45 ppm (t,4H); 3,28 ppm (m; Protonenzahl nicht feststellbar, da Signal durch Signal geringer $H_2O$-Mengen überlagert); 3,38 ppm (t,4H); 3,65 ppm (t,4H); 4,80 ppm (s,OH); 6,25 ppm (s,NH); 6,9 ppm (d,2H); 7,34 ppm (m,4H); 8,08 ppm (s,NH); 9,61 ppm (s,NH); 11,9 ppm (s,OH).

Um dieses Kondensationsprodukt zur Triphendioxazinverbindung zu cyclisieren, trägt man 23 Teile dieses Produktes in 220 Teile 10%igen Oleum bei 20°C langsam ein und rührt das Gemisch noch bis zur vollständigen Lösung nach. Sodann trägt man langsam unter Einhaltung einer Reaktionstemperatur von 20 bis 25°C 11,5 Teile Natriumperoxodisulfat ein, rührt eine Stunde nach, gibt den Ansatz auf Eis, stellt die Suspension mit Calciumcarbonat auf einen pH-Wert zwischen 1 und 1,5, versetzt das Ganze mit Natriumcarbonat bis zu einem pH-Wert von 5,5, filtriert das Calciumsulfat ab, wäscht es mit Wasser und vereinigt die Filtrate. Die erfindungsgemäße Verbindung kann daraus durch Eindampfen oder Sprühtrocknung erhalten werden. Die Aufarbeitung kann aber auch in der Weise erfolgen, daß die in ihrer Säureform beim Hinzugeben des Cyclisierungsansatzes auf Eis ausgefallene erfindungsgemäße Verbindung wieder in Wasser aufgenommen wird, man den pH mit Natriumcarbonat auf einen Wert von 5,5 einstellt und die erfindungsgemäße Verbindung als Alkalimetallsalz (Natriumsalz) durch Eindampfen oder Sprühtrocknung isoliert.

Man erhält das erfindungsgemäße Alkalimetallsalz (Natriumsalz) der Verbindung der Formel

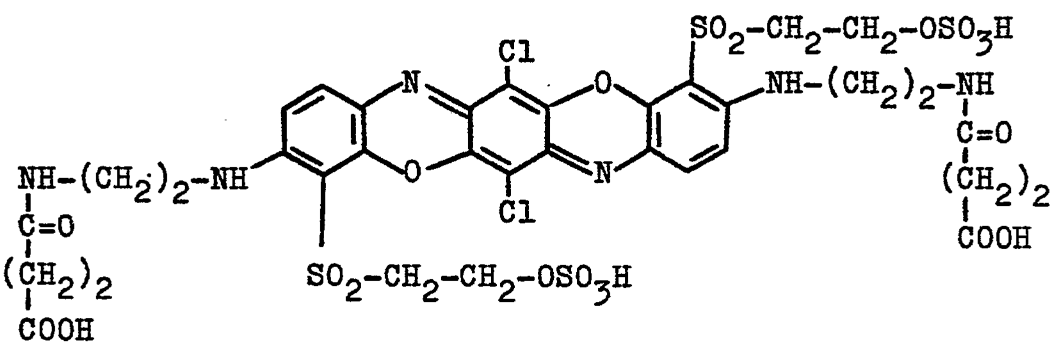

als elektrolytsalzhaltiges (vorwiegend natriumsulfat-haltiges) Pulver (die jeweilige β-Sulfatoethylsulfonyl-Gruppe kann auch in der anderen ortho-Stellung zur Aminogruppe gebunden sein, befindet sich jedoch mit größerer Wahrscheinlichkeit in der in der obigen Formel angegebenen Stellung).

Diese erfindungsgemäße Verbindung besitzt sehr gute faserreaktive Farbstoffeigenschaften ($\lambda_{max}$ = 612 nm). Sie färbt die in der Bechreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen blauen Tönen mit guten Echtheiten, wie insbesondere guter Lichtechtheit der trockenen oder feuchten, wie mit Trinkwasser befeuchteten, Färbungen, guter alkalischen Schweißlichtechtheit, Chlorbadewasserechtheit, Hypochloritechtheit, alkalischer Schweißechtheit, Waschechtheit, Naßliegeechtheit und Säurelagerbeständigkeit.

Beispiel 4

Zur Herstellung einer erfindungsgemäßen Triphendioxazin-Verbindung stellt man, analog den Angaben des Beispieles 3 und unter Verwendung äquivalenter Mengen, eine schwach saure bis neutrale wäßrige Lösung der Anilinverbindung des Beispieles 2c) her und setzt diese Anilinverbindung mit Chloranil um, saugt das ausgefallene chinoide Kondensationsprodukt ab, wäscht es mit etwas Wasser, trocknet es und trägt es sodann bei 20°C in 10%iges Oleum ein. Wie im Beispiel 3 wird die Cyclisierungsreaktion mittels Natriumperoxodisulfat beschleunigt. Nach Beendigung der Cyclisierung und nach Zugabe von Calciumcarbonat und Abfiltration des Calciumsulfats und Einstellung des erhaltenen Filtrats auf einen pH-Wert von 6 isoliert man die erfindungsgemäße Triphendioxazinverbindung als Alkalimetallsalz durch Eindampfen oder Sprühtrocknen. Sie besitzt, in Form der freien Säure geschrieben, die Formel

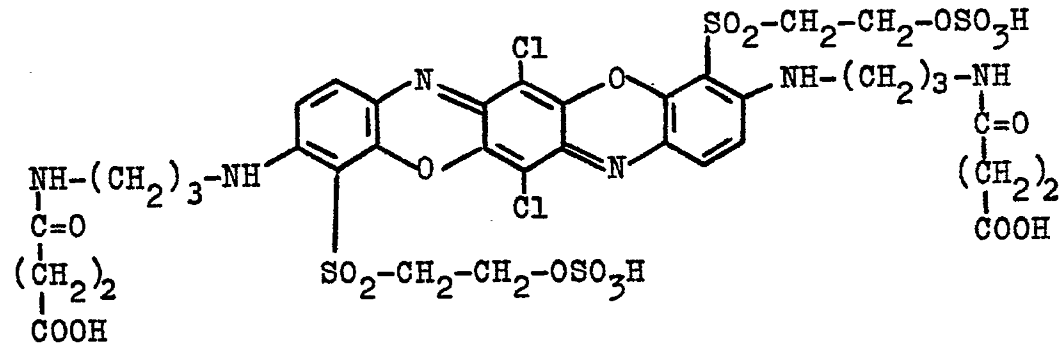

($\lambda_{max}$ = 620 nm).

Die erfindungsgemäße Verbindung besitzt sehr gute faserreaktive Farbstoffeigenschaften und färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen blauen Tönen mit guten Echtheiten, wie insbesondere guter Lichtechtheit der trockenen oder feuchten, wie mit Trinkwasser befeuchteten, Färbungen, guter alkalischen Schweißlichtechtheit, Chlorbadewasserechtheit, Hypochloritechtheit, alkalischer Schweißechtheit, Waschechtheit, Naßliegeechtheit und Säurelagerbeständigkeit.

<u>Beispiele 5 bis 23</u>

In den nachfolgenden Tabellenbeispielen sind weitere erfindungsgemäße Triphendioxazin-Verbindungen mit Hilfe der Formelglieder der allgemeinen Formel (A)

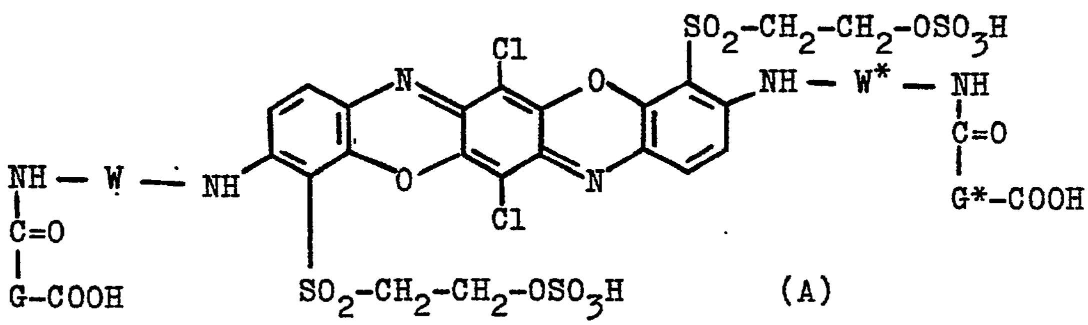

(A)

beschrieben, wobei W den in der Tabelle "spiegelbildlich" geschriebenen Rest von W* und G den dort "spiegelbildlich" geschriebenen Rest von G* darstellen.

Diese erfindungsgemäßen Verbindungen lassen sich in erfindungsgemäßer Weise, so beispielsweise analog dem obigen Ausführungsbeispiel, aus den ersichtlichen Komponenten (Chloranil, einer Verbindung der nachstehenden allgemeinen Formel (3a) und Schwefelsäure oder Oleum) herstellen.

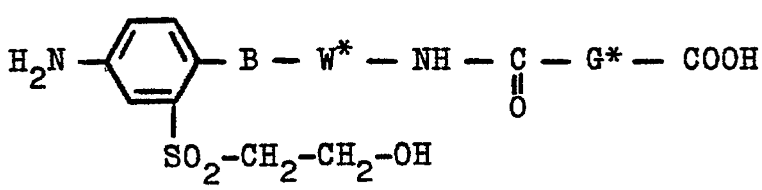

(3a)

Die erfindungsgemäßen Triphendioxazin-Verbindungen besitzen ebenfalls sehr gute faserreaktive Farbstoffeigenschaften und liefern, insbesondere auf Cellulsoefasermaterialien, farbstarke Färbungen und Drucke mit guten Echtheiten in dem in dem jeweiligen Tabellenbeispiel angegebenen Farbton.

| Bsp. | Rest -W*- | Rest -G*- | Farbton |
|---|---|---|---|
| 5 | $-CH_2-CH(CH_3)-$ | 1,2-Ethylen | blau |
| 6 | $-(CH_2)_2-SO_2-(CH_2)_2-$ | dito | blau |
| 7 | $-(CH_2)_2-N(CH_3)-(CH_2)_2-$ | dito | blau |
| 8 | 1,2-Ethylen | $-CH_2-CH(CH_3)-$ | blau |
| 9 | 1,3-Propylen | dito | blau |
| 10 | 1,2-Ethylen | 1,3-Propylen | blau |
| 11 | 1,3-Propylen | dito | blau |
| 12 | $-CH_2-CH(CH_3)-$ | dito | blau |
| 13 | 1,2-Ethylen | 2-Methyl-1,3-propylen | blau |
| 14 | 1,2-Ethylen | $-CH_2-C(CH_3)_2-$ | blau |
| 15 | 1,3-Propylen | dito | blau |
| 16 | $-CH_2-CH(CH_3)-$ | dito | blau |
| 17 | 1,2-Ethylen | 2,2-Dimethyl-1,3-propylen | blau |
| 18 | 1,2-Ethylen | 2-Methyl-2-ethyl-1,3-propylen | blau |
| 19 | 1,2-Ethylen | 1,2-Cyclohexylen | blau |
| 20 | $-CH_2-CH(CH_3)-$ | dito | blau |
| 21 | 1,3-Propylen | dito | blau |
| 22 | 2-Methyl-1,3-propylen | 1,4-Ethylen | blau |
| 23 | $-(CH_2)_2-O-(CH_2)_2-$ | dito | blau |

## Patentansprüche

1. Eine wasserlösliche Triphendioxazin-Verbindung, die der allgemeinen Formel (1)

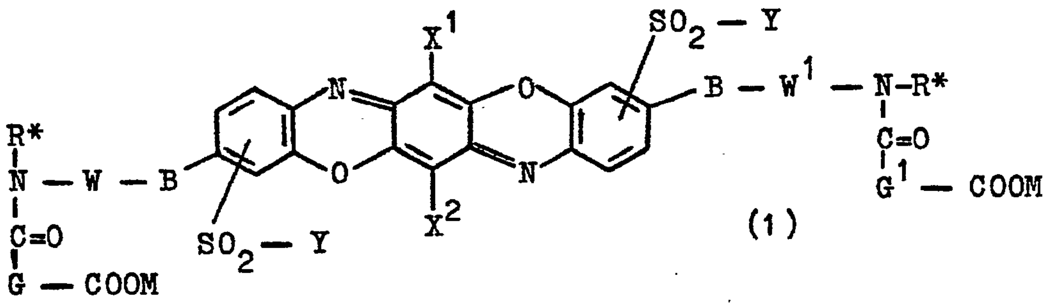

(1)

entspricht, worin bedeuten:
B ist ein Sauerstoff- oder Schwefelatom oder eine Aminogruppe der Formel -NH- oder -N(R')- , in welcher
R' eine Alkylgruppe von 1 bis 6 C-Atomen ist;
R* ist ein Wasserstoffatom oder eine gegebenenfalls substituierte Alkylgruppe von 1 bis 4 C-Atomen oder ein gegebenenfalls substituierter Arylrest;
W ist ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest, wobei die aliphatischen Reste durch Heterogruppen unterbrochen sein können, die aus den Gruppen -O- , -S- , $-SO_2-$ , -CO- , 1,4-Piperidino, -NH- und $-N(R^0)$ , worin $R^0$ eine der Bedeutungen von R' hat oder eine Alkanoylgruppe von 2 bis 5 C-Atomen ist, ausgewählt sind, und
$W^1$ hat eine der für W angegebenen Bedeutungen und ist mit W gleich oder von W verschieden, oder
die Gruppierung $-B-W^1-N(R^*)-$ und die Gruppierung -N(R*)-W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar oder
die Gruppierung $-B-W^1-$ und die Gruppierung -W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar, der mit einem der beiden Stickstoffatome über eine Alkylengruppe von 2 bis 4 C-Atomen mit der Gruppierung $-N(R^*)-CO-G^1-$ bzw. -G-CO-N(R*)- verbunden ist;
G ist eine direkte Bindung oder eine geradkettige oder verzweigte Alkylengruppe von 1 bis 8 C-Atomen oder ein aliphatisch-cycloaliphatischer Rest oder ein cycloaliphatischer Rest, wobei die cycloaliphatischen Reste jeweils solche von 5 bis 8 C0Atomen sind;
$G^1$ hat eine der für G angegebenen Bedeutungen und ist mit G gleich oder von G verschieden;
M ist ein Wasserstoffatom oder ein Alkalimetall, oder das Äquivalent eines Erdalkalimetalls;
$X^1$ ist ein Wasserstoffatom, ein Halogenatom, eine Alkylgruppe von 1 bis 4 C-Atomen, eine Alkoxygruppe von 1 bis 4 C-Atomen, eine Aryloxygruppe oder ein gegebenenfalls subtituierter Arylrest;
$X^2$ ist mit $X^1$ gleich oder von $X^1$ verschieden und hat eine der für $X^1$ angegebenen Bedeutungen;
Y ist die Vinylgruppe oder eine Ethylgruppe, die in β-Stellung einen durch ein Alkali eliminierbaren Substituenten enthält;
die Gruppe $-SO_2-Y$ steht bevorzugt in ortho-Stellung zur Gruppe $-B-W-N(R^*)-CO-G^1-COOM$ bzw. MOOC-G-CO-N(R*)-W-B- gebunden.

2. Verbindung nach Anspruch 1, dadurch gekennzeichnet, daß G oder $G^1$ eine Alkylengruppe von 2 bis 6 C-Atomen oder ein Cyclohexylenrest ist.

3. Verbindung nach Anspruch 1, dadurch gekennzeichnet, daß G oder $G^1$ der 1,2-Ethylen- oder 1,3-Propylen-Rest ist.

4. Verbindung nach Anspruch 1, dadurch gekennzeichnet, daß G oder $G^1$ der 2-Methyl-1,3-propylen-, 2,2-Dimethyl-1,3-propylen-, der 1,2-Cyclohexylen- oder der Isopropylen-Rest ist.

5. Verbindung nach mindestens einem der Ansprüche 1 bis 4, dadurch gekennzeichnet, daß R* ein Wasserstoffatom bedeutet.

6. Verbindung nach mindestens einem der Ansprüche 1 bis 5, dadurch gekennzeichnet, daß B die Gruppe -NH- bedeutet.

7. Verbindung nach mindestens einem der Ansprüche 1 bis 6, dadurch gekennzeichnet, daß W and $W^1$ beide, zueinander gleich oder voneinader verschieden, einen Alkylenrest von 2 bis 4 C-Atomen bedeuten, der durch ein Sauerstoffatom oder eine Gruppe -NH- oder $-N(CH_3)-$ unterbrochen sein kann, oder eine Cycloalkylengruppe von 5 oder 6 C-Atomen mit 1, 2 oder 3 Methylgruppen als Substituenten darstellen oder zwei solche mit einem Alkylenrest von 1 bis 4 C-Atomen verbundene Cycloalkylengruppen oder einen Alkylenrest von 2 bis 6 C-Atomen, der durch eine solche Cycloalkylengruppe unterbrochen ist, darstellen.

8. Verbindung nach mindestens einem der Ansprüche 1 bis 6, dadurch gekennzeichnet, daß W und $W^1$ beide für den 1,2-Ethylen-, 1,3-Propylen-, 1,4-Butylen- oder den Isopropylen-Rest stehen.

9. Verbindung nach mindestens einem der Ansprüche 1 bis 8, dadurch gekennzeichnet, daß beide Y eine β-Sulfatoethyl-Gruppe bedeuten.

10. Verbindung nach mindestens einem der Ansprüche 1 bis 9, dadurch gekennzeichnet, daß $X^1$ und $X^2$ beide ein Chlor- oder ein Bromatom bedeuten.

11. Verbindung nach mindestens einem der Ansprüche 1 bis 9, dadurch gekennzeichnet, daß $X^1$ und $X^2$ beide ein Chloratom bedeuten.

12. Verbindung nach Anspruch 1 der allgemeinen Formel (1a)

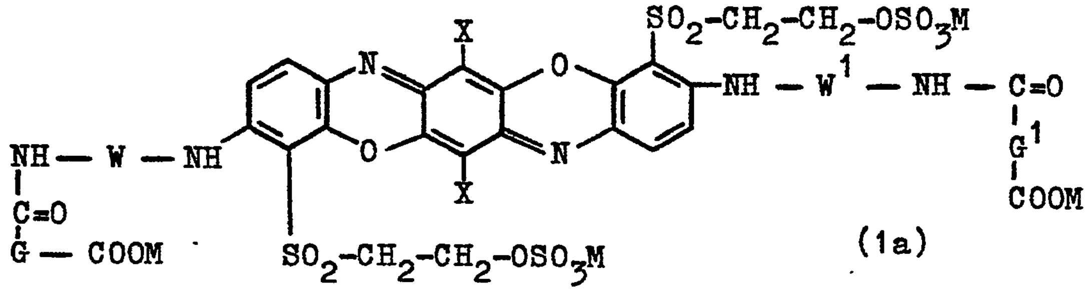

(1a)

in welcher
W und $W^1$ beide eine Alkylengruppe von 2 bis 6 C-Atomen bedeuten,
X jedes für ein Bromatom oder insbesondere bevorzugt ein Chloratom steht,
M ein Wasserstoffatom oder ein Alkalimetall ist und
G und $G^1$ beide eine Alkylengruppe von 2 bis 6 C-Atomen bedeuten.

13. Verfahren zur Herstellung einer in Anspruch 1 genannten und definierten Verbindung der allgemeinen Formel (1), dadurch gekennzeichnet, daß man eine Verbindung der allgemeinen Formel (2)

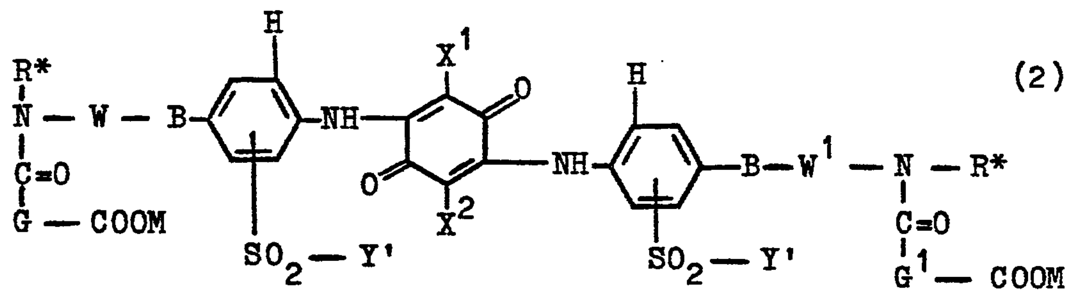

(2)

(in welcher Y′ die Vinylgruppe, die β-Hydroxyethyl-Gruppe oder eine Ethylgruppe, die in β-Stellung einen durch ein Alkali eliminierbaren Substituenten, wie einen der für Y genannten, enthält, bevorzugt die β-Hydroxyethyl-Gruppe ist und B, G, $G^1$, R*, W, $W^1$, $X^1$ und $X^2$ die in Anspruch 1 genannten Bedeutungen haben, wobei substituierte Alkylgruppen in diesen Resten auch hydroxysubstituierte Alkylgruppen sein können, die Gruppen $-SO_2-Y'$ bevorzugt in ortho-Stellung zur Gruppe B gebunden sind und die Benzolkerne in einer der ortho-Stellungen zur angegebenen Aminogruppe -NH- nicht substituiert sein dürfen)
in saurem Medium und vorzugsweise in Gegenwart eines Oxidationsmittels zum Triphendioxazin cyclisiert und gegebenenfalls während der Cyclisierung oder nach der Cyclisierung eine oder mehrere Sulfogruppen in einen Arylrest einführt.

14. Verfahren nach Anspruch 13, dadurch gekennzeichnet, daß man gleichzeitig mit der Cyclisierung oder erst nach der Cyclisierung gegebenenfalls vorhandene Hydroxyalkylgruppen in Sulfatoalkyl- oder Phosphatoalkylgruppen überführt.

15. Verfahren nach Anspruch 13, dadurch gekennzeichnet, daß man die Cyclisierung in Schwefelsäure oder Schwefeltrioxid enthaltender Schwefelsäure mit einem Alkalipersulfat durchführt.

16. Verfahren nach Anspruch 13, dadurch gekennzeichnet, daß man die Cyclisierung in 10 bis 50%igem Oleum bei 0 bis 40°C in Gegenwart einer katalytischen Menge Jod oder einer Jodverbindung durchführt.

17. Verwendung einer in Anspruch 1 genannten und definierten Verbindung der allgemeinen Formel (1) zum Färben (einschließlich Bedrucken) von hydroxy- und/oder carbonamidgruppenhaltigem Material, insbesondere Fasermaterial.

18. Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder in das Material einbringt und ihn mittels Wärme und/oder mit Hilfe eines alkalisch wirkenden Mittels fixiert, dadurch gekennzeichnet, daß man als Farbstoff eine Verbindung der in Anspruch 1 genannten und definierten allgemeinen Formel (1) einsetzt.

19. Eine Verbindung der allgemeinen Formel (9)

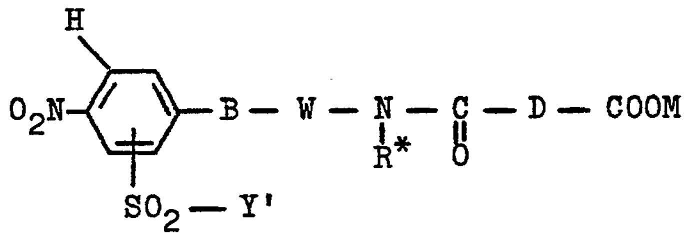

(9)

in welcher
B ein Sauerstoff oder Schwefelatom oder eine Aminogruppe der Formel -NH- oder -N(R')- ist, in welcher R' eine Alkylgruppe von 1 bis 6 C-Atomen bedeutet,
W ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest ist, wobei die aliphatischen Reste durch Heterogruppen unterbrochen sein können, die aus den Gruppen -O- , -S- , -$SO_2$- , -CO- , 1,4-Piperidino, -NH- und -N($R^0$)- , worin $R^0$ eine der Bedeutungen von R' hat oder eine Alkanoylgruppe von 2 bis 5 C-Atomen sit, ausgewählt sind,
R* ein Wasserstoffatom oder eine gegebenenfalls substituierte Alkylgruppe von 1 bis 4 C-Atomen oder ein gegebenenfalls substituierter Arylrest ist,
Y' die Vinylgruppe oder eine Ethylgruppe bedeutet, die in β-Stellung einen durch ein Alkali eliminierbaren Substituenten enthält, oder die β-Hydroxyethyl-Gruppe ist,
D ist ein geradkettiger oder verzweigter Alkenylenrest von 2 bis 8 C-Atomen oder Alk-dien-ylen-Rest von 4 bis 8 C-Atomen und
M ist ein Wasserstoffatom, ein Alkalimetall oder das Äquivalent eines Erdalkalimetalls.

20. Eine Verbindung der allgemeinen Formel (8)

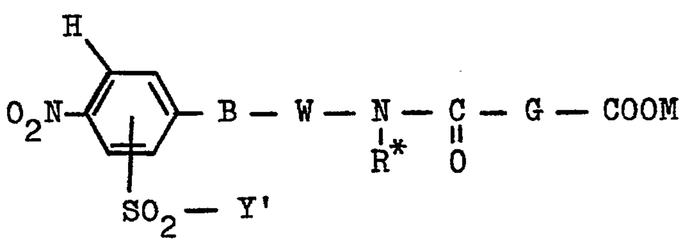

(8)

in welcher
B ein Sauerstoff oder Schwefelatom oder eine Aminogruppe der Formel -NH- oder -N(R')- ist, in welcher R' eine Alkylgruppe von 1 bis 6 C-Atomen bedeutet,
W ein bivalenter, aliphatischer oder gegebenenfalls durch Alkyl von 1 bis 4 C-Atomen substituierter ($C_5$–$C_{10}$)-cycloaliphatischer oder gegebenenfalls durch Alkyl von 1 bis 4 C-Atomen substituierter aliphatisch-($C_5$–$C_8$)-cycloaliphatischer Rest ist, wobei die aliphatischen Reste durch Heterogruppen unterbrochen sein können, die aus den Gruppen -O-, -S-, -$SO_2$-, -CO-, 1,4-Piperidino, -NH- und -N($R^0$)-, worin $R^0$ eine der Bedeutungen von R' hat oder eine Alkanoylgruppe von 2 bis 5-Atomen ist, ausgewählt sind,
R* ein Wasserstoffatom oder eine gegebenenfalls substituierte Alkylgruppe von 1 bis 4 C-Atomen oder ein gegebenenfalls substituierter Arylrest ist,
Y' die Vinylgruppe oder eine Ethylgruppe bedeutet, die in β-Stellung einen durch ein Alkali eliminierbaren Substituenten enthält, oder die β-Hydroxyethyl-Gruppe ist,
G ist eine direkte Bindung oder eine geradkettige oder verzweigte Alkylengruppe von 1 bis 8 C-Atomen oder ein aliphatisch-cycloaliphatischer Rest oder ein cycloaliphatischer Rest, wobei die cycloaliphatischen Reste jeweils solche von 5 bis 8 C-Atomen sind und
M ist ein Wasserstoffatom, ein Alkalimetall oder das Äquivalent eines Erdalkalimetalls.

21. Eine Verbindung der allgemeinen Formel (3)

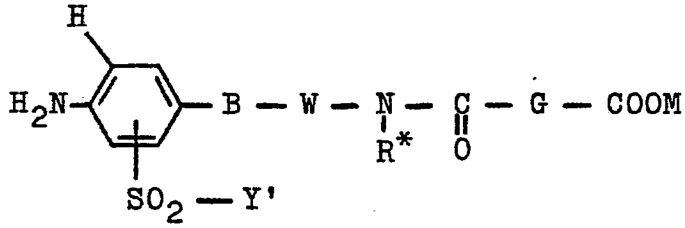

(3)

in welcher
B ein Sauerstoff oder Schwefelatom oder eine Aminogruppe der Formel -NH- oder -N(R')- ist, in welcher R' eine Alkylgruppe von 1 bis 6 C-Atomen bedeutet,
W ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest ist, wobei die aliphatischen Reste durch Heterogruppen unterbrochen sein können, die aus den Gruppen -O- , -S- , $-SO_2-$ , -CO- , 1,4-Piperidino, -NH- und -N($R^0$)- , worin $R^0$ eine der Bedeutungen von R' hat oder eine Alkanoylgruppe von 2 bis 5 C-Atomen ist, ausgewählt sind,
R* ein Wasserstoffatom oder eine gegebenenfalls substituierte Alkylgruppe von 1 bis 4 C-Atomen oder ein gegebenenfalls substituirter Arylrest ist,
Y' die Vinylgruppe oder eine Ethylgruppe bedeutet, die in β-Stellung einen durch ein Alkali eliminierbaren Substituenten enthält, oder die β-Hydroxyethyl-Gruppe ist,
G ist eine direkte Bindung oder eine geradkettige oder verzweigte Alkylengruppe von 1 bis 8 C-Atomen oder ein aliphatisch-cycloaliphatischer Rest oder ein cycloaliphatischer Rest, wobei die cycloaliphatischen Reste jeweils solche von 5 bis 8 C-Atomen sind und
M ist ein Wasserstoffatom, ein Alkalimetall oder das Äquivalent eines Erdalkalimetalls.

22. Verbindung nach Anspruch 19, 20 oder 21, dadurch gekennzeichnet, daß B eine Gruppe der Formel -NH- ist.

23. Verfahren zur Herstellung einer in Anspruch 19, 20 oder 21 angegebenen und definierten Verbindung der allgemeinen Formel (9) bzw. (8) bzw. (3), dadurch gekennzeichnet, daß man eine Verbindung der allgemeinen Formel (5)

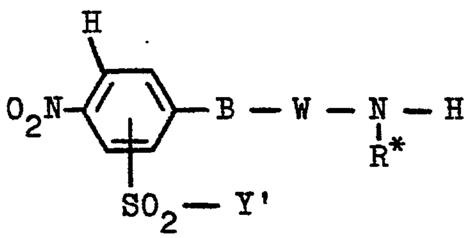

(5)

in welcher Y', B, W und R* die in Anspruch 19 bzw. Anspruch 20 bzw. Anspruch 21 genannten Bedeutungen haben, mit einem Anhydrid der allgemeinen Formel (6) oder (7)

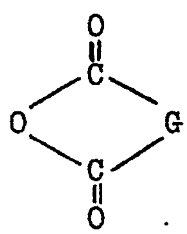

(6)

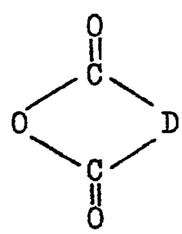

(7)

umsetzt und gegebenenfalls in der so erhaltenen Verbindung die Nitrogruppe und eine gegebenenfalls vorhandene olefinische Bindung reduziert.

24. Verwendung einer Verbindung der allgemeinen Formel (9) von Anspruch 19 oder einer Verbindung der allgemeinen Formel (8) von Anspruch 20 oder einer Verbindung der allgemeinen Formel (3) von Anspruch 21 zur Synthese von Farbstoffen, insbesondere von Triphendioxazin-Verbindungen der allgemeinen Formel (1) gemäß Anspruch 1.

## Claims

1. A water-soluble triphendioxazine compound which conforms to the general formula (1)

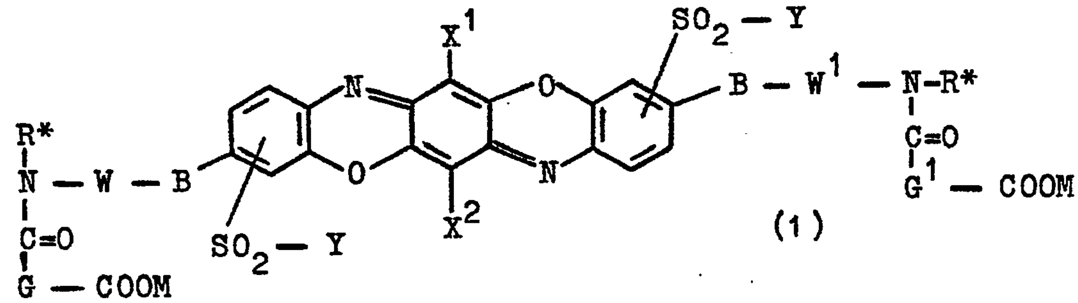

(1)

in which the meanings are

B is an oxygen or sulfur atom or an amino group of the formula –NH– or –N(R′)–, in which R′, is an alkyl group of 1 to 6 carbon atoms; R* is a hydrogen atom or an optionally substituted alkyl group of 1 to 4 carbon atoms or an optionally substituted aryl radical; W is a bivalent, aliphatic or optionally $C_1$–$C_4$-alkyl-substituted ($C_5$–$C_{10}$)-cycloaliphatic or optionally $C_1$–$C_4$-alkyl-substituted aliphatic-($C_5$–$C_8$)-cycloaliphatic radical, which aliphatic radicals can be interrupted by hetero groups which are selected from the groups –O–, –S–, –$SO_2$–, –CO–, 1,4-piperidino, –NH–, and –N(R°), where R° has one of the meanings of R′ or is an alkanoyl group of 2 to 5 carbon atoms, and

$W_1$ has one of the meanings indicated for Wand is identical to or different from W, or the grouping –B–$W_1$–N(R*)– and the grouping –N(R*)–W–B–, identical to or different from each other, each represent together the bivalent radical of a five- or six-membered saturated heterocycle which contains two nitrogen atoms or the grouping –B–$W_1$– and the grouping –W–B–, identical to or different from each other, each represent together the bivalent radical of a five- or sixmembered saturated heterocycle which contains two nitrogen atoms and which is bonded by one of the two nitrogen atoms via an alkylene group of 2 to 4 carbon atoms to the grouping –N(R*)–CO–$G_1$– or –G–CO–N(R*)–; G is a direct bond or a straight-chain or branched alkylene group of 1 to 8 carbon atoms or an aliphatic-cycloaliphatic radical or a cycloaliphatic radical, the cycloaliphatic radicals being in each case those of 5 to 8 carbon atoms; $G^1$ has one of the meanings indicated for G and is identical to or different from G; M is a hydrogen atom or an alkali metal or one equivalent of an alkaline earth metal; $X^1$ is a hydrogen atom, a halogen atom, an alkyl group of 1 to 4 carbon atoms, an alkoxy group of 1 to 4 carbon atoms, an aryloxy group or an optionally substituted aryl radical; $X^2$ is identical to or different from $X_1$ and has one of the meanings indicated for $X^1$; Y is the vinyl group or an ethyl group which contains in the β-position an alkali-eliminable - substituent;

the group –$SO_2$–Y is preferably bonded in the orthoposition relative to the group –B–W–N(R*)CO–$G_1$–COOM or MOOC–G–CO–N(R*)–W–B–.

2. The compound as claimed in claim 1, wherein G or $G^1$is an alkylene group of 2 to 6 carbon atoms or a cyclohexylene radical.

3. The compound as claimed in claim 1, wherein G or $G^1$is the 1,2-ethylene or 1,3-propylene radical.

4. The compound as claimed in claim 1, wherein G or $G^1$ is the 2-methyl-1,3-propylene, 2,2-dimethyl-1, 3-propylene, the 1,2-cyclohexylene or the isopropylene radical.

5. The compound as claimed in one or more of claims 1 to 4, wherein R* denotes a hydrogen atom.

6. The compound as claimed in one or more of claims 1 to 5, wherein B denotes the group -NH-.

7. The compound as claimed in one or more of claims 1 to 6, wherein W and $W^1$ both, identical to or different from each other, denote an alkylene radical of 2 to 4 carbon atoms which can be interrupted by; an oxygen atom or a group –NH– or –N($CH_3$)–, or represent a cycloalkylene group of 5 or 6 carbon atoms having 1, 2 or 3 methyl groups as substituents or represent two such cycloalkylene groups bonded to an alkylene radical of 1 to 4 carbon atoms or represent an alkylene radical of 2 to 6 carbon atoms which is interrupted by such a cycloalkylene group.

8. The compound as claimed in one or more of claims 1 to 6, wherein W and $W^1$ both stand for the 1, 2-ethylene, 1,3-propylene, 1,4-butylene or the isopropylene radical.

9. The compound as claimed in one or more of claims 1 to 8, wherein both the is denote a β-sulfatoethyl group.

10. The compound as claimed in one or more of claims 1 to 9, wherein $X^1$and $X^2$ both denote a chlorine or a bromine atom.

11. The compound as claimed in one or more of claims 1 to 9, wherein $X^1$and $X^2$ both denote a chlorine atom.

12. The compound as claimed in claim 1 of the general formula (1a)

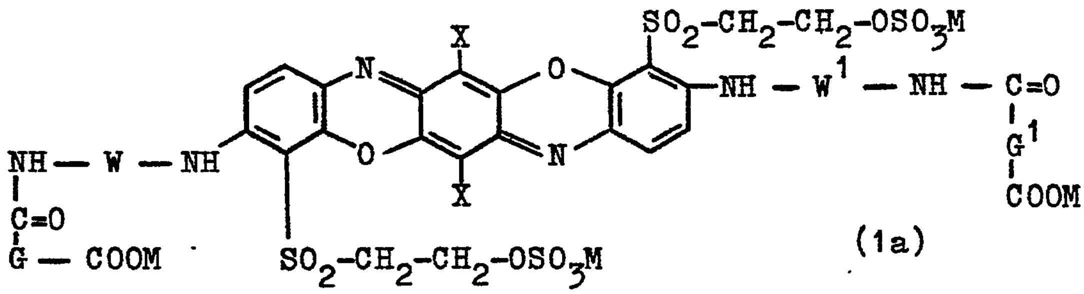

(1a)

in which W and $W^1$ both denote an alkylene group of 2 to 6 carbon atoms, X in each case stands for a bromine atom or particularly preferably a chlorine atom, M is a hydrogen atom or an alkali metal and G and $G^1$ both denote an alkylene group of 2 to 6 carbon atoms.

13. A process for preparing a compound mentioned and defined in claim 1, of the general formula (1), which comprises cyclizing a compound of the general formula (2)

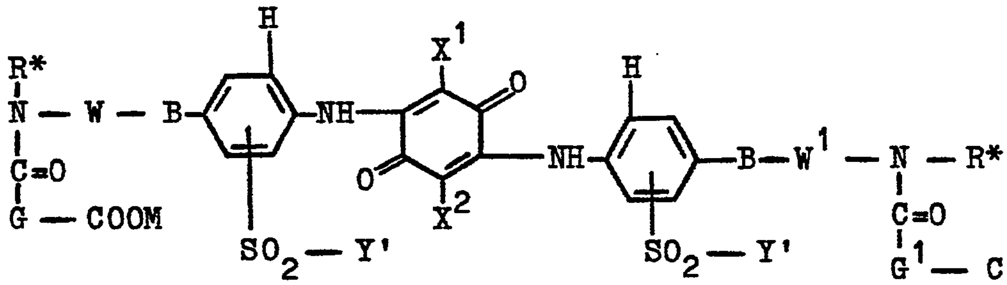

(2)

(in which Y' is the vinyl group, the β-hydroxyethyl group or an ethyl group which contains in the β-position an alkali-eliminable substituent, such as one of those substituents mentioned for Y, preferably is the β-hydroxyethyl group, and B, G, $G^1$, R*, W, $W^1$, $X^1$ and $X^2$ have the meanings mentioned in claim 1, where substituted alkyl groups in these radicals can also be hydroxyl-substituted alkyl groups, the groups $-SO_2-Y'$ are preferably bonded in the orthoposition relative to the group B, and the benzene nuclei must not be substituted in any of the orthopositions relative to the amino group -NH- shown) in an acid medium and preferably in the presence of an oxidizing agent to the triphendioxazine and if desired during the cyclization or after the cyclization introducing one or more sulfo groups into an aryl radical.

14. The process as claimed in claim 13, wherein any hydroxyalkyl groups present are converted into sulfatoalkyl groups or phosphatoalkyl groups simultaneously with the cyclization or only after the cyclization.

15. The process as claimed in claim 13, wherein the cyclization is carried out in sulfuric acid or $SO_3$-containing sulfuric acid with an alkali metal persulfate.

16. The process as claimed in claim 13, wherein the cyclization is carried out in 10 to 50% strength oleum at 0 to 40°C in the presence of a catalytic amount of iodine or an iodine compound.

17. Use of the compound mentioned and defined in claim 1, of the general formula (1), for dyeing (including printing) hydroxyl- and/or carboxamido-containing material, in particular fiber material.

18. A process for dyeing (including printing) hydroxyl- and/or carboxamido-containing material, in particular fiber material, wherein a dye is applied to or incorporated into the material and fixed by means of heat and/or with the aid of an alkaline agent, which comprises using as the dye a compound of the general formula (1) mentioned and defined in claim 1.

19. A compound of the general formula (9)

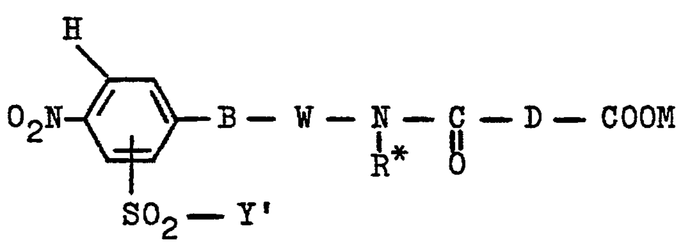

(9)

in which B is an oxygen or sulfur atom or an amino group of the formula –NH– or –N(R′)–, in which R′ denotes an alkyl group of 1 to 6 carbon atoms, W is a bivalent, aliphatic or optionally $C_1$–$C_4$-alkyl-substituted ($C_5$–$C_{10}$)-cycloaliphatic or optionally $C_1$–$C_4$-alkyl-substituted aliphatic($C_5$–$C_8$)-cycloaliphatic radical, which aliphatic radicals can be interrupted by hetero groups which are selected from the groups –O–, –S–, –$SO_2$–, –CO–, 1,4-piperidino, –NH–, and –N(R°)–, where R° has one of the meanings of R′ or is an alkanoyl group of 2 to 5 carbon atoms, R* is a hydrogen atom or an optionally substituted alkyl group of 1 to 4 carbon atoms or an optionally substituted aryl radical, Y′ denotes the vinyl group or an ethyl group which contains an alkali-eliminable substituent in the β-position, or is the β-hydroxyethyl group, D is a straight-chain or branched alkenylene radical of 2 to 8 carbon atoms or alkdienylene radical of 4 to 8 carbon atoms and M is a hydrogen atom, an alkali metal or one equivalent of an alkaline earth metal.

20. A compound of the general formula (8)

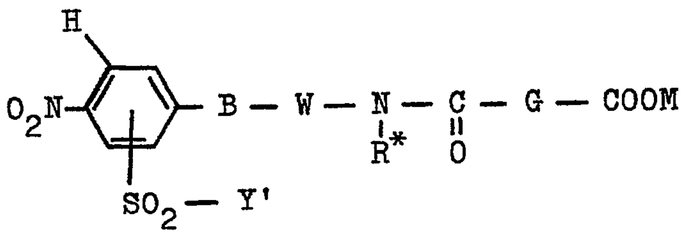

(8)

in which B is an oxygen or sulfur atom or an amino group of the formula –NH– or –N(R′)–, in which R′ denotes an alkyl group of 1 to 6 carbon atoms, W is a bivalent, aliphatic or optionally $C_1$–$C_4$-alkyl-substituted ($C_5$–$C_{10}$)-cycloaliphatic or optionally $C_1$–$C_4$-alkyl-substituted aliphatic-($C_5$–$C_8$)-cycloaliphatic radical, which aliphatic radicals can be interrupted by hetero groups which are selected from the groups –O–, –S–, –$SO_2$–, –CO–, 1,4-piperidino, –NH–, and –N(R°)–, where R° has one of the meanings of R′, or is an alkanoyl group of 2 to 5 carbon atoms, R* is a hydrogen atom or an optionally substituted alkyl group of 1 to 4 carbon atoms or an optionally substituted aryl radical, Y′ denotes the vinyl group or an ethyl group which contains an alkali-eliminable substituent in the β-position, or is the β-hydroxyethyl group, G is a direct bond or a straight-chain or branched alkylene group of 1 to 8 carbon atoms or an aliphatic-cycloaliphatic radical which cycloaliphatic radicals are in each case those of 5 to 8 carbon atoms, and M is a hydrogen atom, an alkali metal or one equivalent of an alkaline earth metal.

21. A compound of the general formula (3)

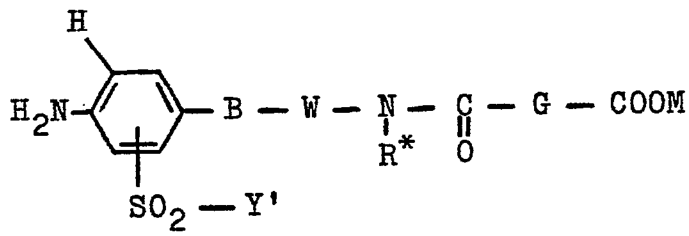

(3)

in which B is an oxygen or sulfur atom or an amino group of the formula –NH– or –N(R′)–, in which R′ denotes an alkyl group of 1 to 6 carbon atoms, W is a bivalent, aliphatic or optionally $C_1$–$C_4$–alkyl-substituted ($C_5$–$C_{10}$)-cycloaliphatic or optionally $C_1$–$C_4$-alkyl-substituted aliphatic($C_5$–$C_8$)-cycloaliphatic radical, which aliphatic radicals can be interrupted by hetero groups which are selected from the groups –O–, –S–, –$SO_2$–, –CO–, 1,4-piperidino, –NH–, and –N(R°)–, where R° has one of the meanings of R′, or is an alkanoyl group of 2 to 5 carbon atoms, R* is a hydrogen atom or an optionally substituted alkyl group of 1 to 4 carbon atoms or an optionally substituted aryl radical, Y′, denotes the vinyl group or an ethyl group which contains an alkali-eliminable substituent in the β-position, or is the β-hydroxyethyl group, G is a direct bond or a straight-chain or branched alkylene group of 1 to 8 carbon atoms or an aliphatic-cycloaliphatic radical or a cycloaliphatic radical, which cycloaliphatic radicals are in each case those of 5 to 8 carbon atoms, and M is a hydrogen atom, an alkali metal or one equivalent of an alkaline earth metal.

22. The compound as claimed in claim 19, 20 or 21, wherein B is a group of the formula –NH–.

23. A process for preparing a compound indicated and defined in claim 19, 20 or 21, of the general formula (9) or (8) or (3), which comprises reacting a compound of the general formula (5)

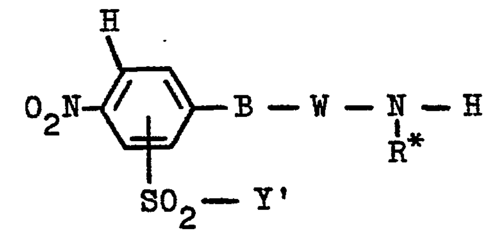

(5)

in which Y′, B, W and R* have the meanings mentioned in claim 19, claim 20 or claim 21, with an anhydride of the general formula (6) or (7)

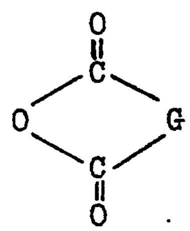

(6)

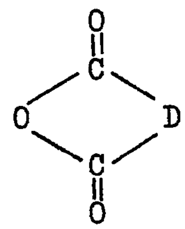

(7)

and if desired reducing the nitro group in the compound thus obtained and any olefinic bond present.

24. Use of a compound of the general formula (9) of claim 19 or of a compound of the general formula (8) of claim 20 or of a compound of the general formula (3) of claim 21, for synthesizing dyes, in particular triphendioxazine compounds of the general formula (1) as claimed in claim 1.

## Revendications

1. Les composés triphénodioxaniques de formule générale (1) ci-dessous:

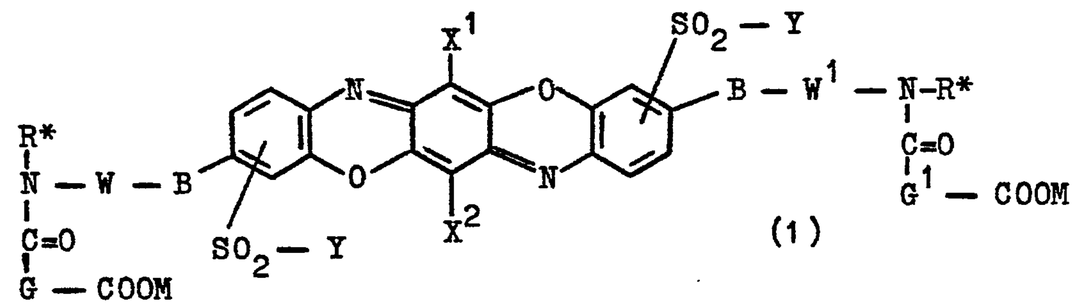

(1)

dans laquelle:

B représente un atome d'oxygène ou de soufre ou un groupe amino –NH– ou –N(R′)–, R′ étant un alkyle en $C_1$–$C_6$;

R* représente un atome d'hydrogène ou bien un alkyle en $C_1$–$C_4$ ou un aryle éventuellement substitués; W un radical bivalent, aliphatique ou ($C_5$–$C_{10}$)-cycloaliphatique éventuellement substitué par un alkyle en $C_1$–$C_4$ ou un encore un radical aliphatico-($C_5$–$C_8$)-cyclo-aliphatique éventuellement substitué par un alkyle en $C_1$–$C_4$, les radicaux aliphatiques pouvant être interrompus par des hétérogroupes pris parmi –O–, –S–, –SO2–, –CO–, les groupes 1,4-pipéridino, –NH– et –N(R°), R° ayant l'une des significations indiquées par R′ ou étant un alcanoyle en $C_2$ à $C_5$, et a $W^1$ l'une des spécifications indiquées pour W et est identique à W ou différent de W, ou bien les groupements –B–$W^1$–N(R*)– et –N(R*)–W–B–, identiques ou différents l'un de l'autre, représentent chacun le radical bivalent d'un hétérocycle pentogonal ou hexagonal saturé à deux atomes d'azote, ou encore les groupements –B–$W^1$– et –W–B–, identiques ou différents l'un de l'autre, représentent chacun le radical bivalent d'un hétérocycle pentogonal ou hexagonal saturé à deux atomes d'azote, lié avec l'un des atomes d'azote par un alkylène en $C_2$–$C_4$ au groupement –N(R*)–CO–$G^1$– ou –G–CO–N(R*)–; G désigne une liaison directe ou un alkylène linéaire ou ramifié en $C_1$–$C_8$, ou encore un radical divalent aliphatique-cycloaliphatique ou cyclo-aliphatique avec chacun de 5 à 8 atomes de carbone dans le cycle; $G^1$ a l'une des significations indiquées pour G et est identique à G ou différent de G; M désigne un atome d'hydrogène ou de métal alcalin ou l'équivalent d'un métal alcaline-terreux; $X^1$ est un atome d'hydrogène ou d'un halogène ou bien un alkyle en $C_1$ à $C_4$ ou un alcoxy en $C_1$–$C_4$ ou encore un groupe aryloxy ou un groupe aryle éventuellement substitué; $X^2$ est

identique à $X^1$ ou différent de $X^1$ et a l'une des significations données pour $X^1$; Y est le groupe vinyle ou un groupe éthyle, avec à la position β un substituant pouvant s'éliminer par un alcali; et les groupes $-SO_2-Y$ sont de préférence en une position ortho par rapport aux groupes respectifs $-B-W-N(R^*)-CO-G^1-COOM$ et $MOOC-G-CO-N(R^*)-W-B-$.

2. Composé selon la revendication 1, caractérisé en ce que G ou $G^1$ est un alkylène en $C_2$ à $C_6$ ou le groupe cyclohexylène.

3. Composé selon la revendication 1, caractérisé en ce que G ou $G^1$ est le radical 1,2-éthylène ou 1,3-propylène.

4. Composé selon la revendication 1, caractérisé en ce que G ou $G^1$ est le radical 2-méthyl-1,3-propylène, 2,2-diméthyl-1,3-propylène, 2,2-diméthyl-1,3-propylène, 1,2-cyclohexylène ou isopropylène.

5. Composé selon une ou plusieurs des revendications 1 à 4, caractérisé en ce qu R* est un atome d'hydrogène.

6. Composé selon une ou plusieurs des revendications 1 à 5, caractérisé en ce que B est le groupe –NH–.

7. Composé selon une ou plusieurs des revendications 1 à 6, caractérisé en ce que W et $W^1$, qui peuvent être identiques ou différents l'un de l'autre, sont des alkylènes en $C_2$ à $C_4$ pouvant être interrompus par un atome d'oxygène ou un groupe –NH– ou $-N-CH_3)-$, ou bien des cycloalkylènes en $C_5$ ou $C_6$ avec comme substituants 1, 2 ou 3 groupes méthyle ou encore deux de tels groupes cycloalkylènes liés à un alkylène en $C_1-C_4$ ou des alkylènes en $C_2$ à $C_6$ interrompus par un tel groupe cyclo-alkylène.

8. Composé selon une ou plusieurs des revendications 1 à 6, caractérisé en ce que W et $W^1$ sont chacun le radical 1,2-éthylène, 1,3-propylène, 1,4-butylène ou isopropylène.

9. Composé selon une ou plusieurs des revendications 1 à 8, caractérisé en ce que les deux Y sont des groupes β-sulfatoéthyle.

10. Composé selon une ou plusieurs des revendications 1 à 9, caractérisé en ce que $X^1$ et $X^2$ sont des atomes de chlore ou de brome.

11. Composé selon une ou plusieurs des revendications 1 à 9, caractérisé en ce que $X^1$ et $X^2$ sont des atomes de chlore.

12. Composé selon la revendication 1, de formule générale (1a) ci-dessous:

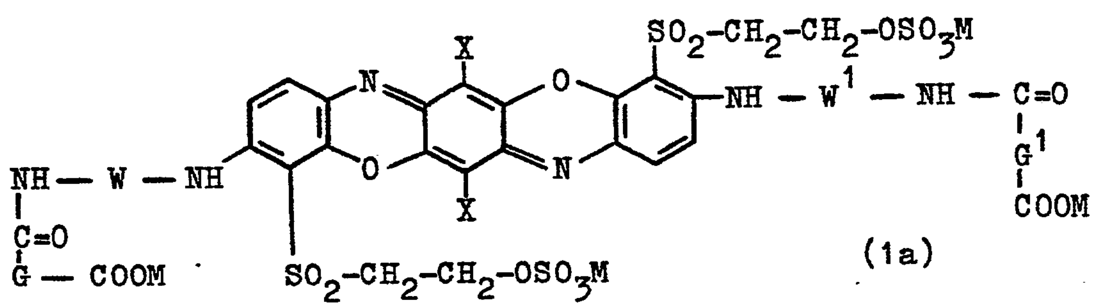

(1a)

dans laquelle W et $W^1$ sont des alkylènes en $C_2$ à $C_6$, les deux X sont des atomes de brome ou en particulier des atomes de chlore, M est un atome d'hydrogène ou d'un métal alcalin et G et $G^1$ sont des alkylènes en $C_2$ à $C_6$.

13. Procédé de préparation des composés de formule générale (1) selon la revendication 1, procédé caractérisé en ce que l'on cyclise en une triphénodioxazine un composé de formule générale (2)

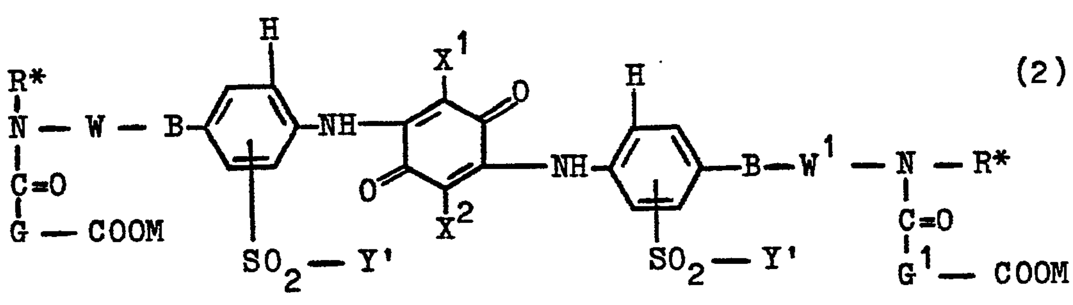

(2)

(dans laquelle Y' est le groupe vinyle ou β-hydroxyéthyle ou un groupe éthyle avec en position β un substituant éliminable par un alcali, tel que l'un de ceux qui ont été indiqués pour Y, mais de préférence le groupe β-hydroxyéthyle, et les autres symboles ont les significations précédemment données, les alkyles substitués de ces radicaux pouvant être aussi des hydroxy-alkyles, les groupes $-SO_2-Y'$ sont de préférence en une position ortho par rapport aux groupes respectifs B, et les cycles benzéniques n'ont

pas de substiuants à l'une des positions ortho par rapport au groupe amino –NH–), en milieu acide et de préférence en présence d'un agent d'oxydant, et le cas échéant au cours de la cyclisation ou après celle-ci, on fixe un ou plusieurs groupes sulfoniques sur un radical aryle.

14. Procédé selon la revendication 13, caractérisé en ce que simultanément à la cyclisation ou seulement après celle-ci on transforme des groupes hydroxyalkyliques éventuellement présents en groupes sulfatoalkyliques ou phosphatoalkyliques.

15. Procédé selon la revendication 13, caractérisé en ce que l'on effectue la cyclisation dans de l'acide sulfurique contenant éventuellement du trioxyde de soufre avec un persulfate de métal alcalin.

16. Procédé selon la revendication 13, caractérisé en ce que l'on effectue la cyclisation dans de l'oléum de 10 à 50% à une température de 0 à 40°C en présence d'une quantité catalytique d'iode ou d'un composé d'iode.

17. Emploi des composés de formule générale (1) selon la revendication 1 pour la teinture (ce qui comprend également l'impression) de matières, en particulier de matières fibreuses, à groupes hydroxyliques et/ou carboxamides.

18. Procédé de teinture (ce qui comprend aussi l'impression) de matières, en particulier de matières fibreuses, à groupes hydroxyliques et/ou carboxamides, par application d'un colorant sur la matière ou incorporation du colorant dans celle-ci et fixage du colorant par la chaleur et/ou avec un agent alcalin, procédé caractérisé en ce que l'on utilise comme colorant un composé de formule générale (1) selon la revendication 1.

19. Un composé de formule générale (9)

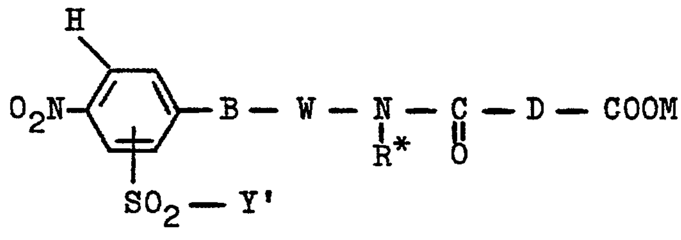

(9)

dans laquelle B est un atome d'oxygène ou de soufre ou un groupe amino –NH– ou –N(R')–, R' étant un alkyle en $C_1$–$C_6$, W est un radical bivalent, aliphatique, ou cycloaliphatique en $C_5$–$C_{10}$ avec éventuellement comme substituant un alkyle en $C_1$–$C_4$, ou encore aliphatique-($C_5$ en $C_8$)-cycloaliphatique avec éventuellement comme substiuant également un alkyle en $C_1$–$C_4$, les radicaux aliphatiques pouvant être interrompus par des hétérogroupes tels que –O–, –S–, $-SO_2-$, –CO–, 1,4-pipéridino, –NH– et –N(R°)–, R° ayant l'une des siginifications de R' ou étant un alcanoyle en $C_2$ à $C_5$, R* est un atome d'hydrogène ou bien un alkyle en $C_1$–$C_4$ ou un aryle éventuellement substiués, Yé le groupe vinyle ou un groupe éthyle avec à la position β un substiuant éliminable par un alcali, ou le groupe β-hydroxyéthyle, D un alcénylène en $C_2$ à $C_8$ ou un alc-diénylène en $C_4$ à $C_8$, chacun à chaîne linéaire ou ramifiée, et M est un atome d'hydrogène, un métal alcalin ou l'équivalent d'un métal alcalino-terreux.

20. Un composé de formule générale (8)

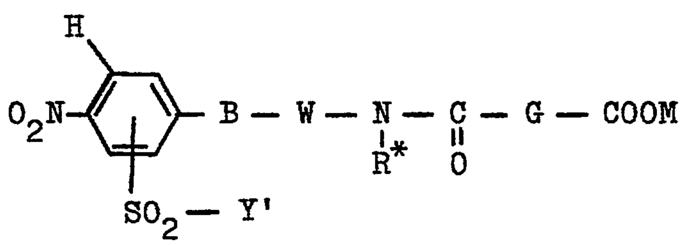

(8)

dans laquelle

B est un atome d'oxygène ou de soufre ou un groupe amino –NH– ou –N(R')–, R' étant un alkyle en $C_1$–$C_6$, W est un radical bivalent, aliphatique, ou cycloaliphatique en $C_5$–$C_{10}$ avec éventuellement comme substituant un alkyle en $C_1$–$C_4$, ou encore aliphatique-($C_5$ en $C_8$)-cycloaliphatique avec éventuellement comme substituant également un alkyle en $C_1$–$C_4$, les radicaux aliphatiques pouvant être interrompus par des hétérogroupes tels que –O–, –S–, $-SO_2-$, –CO–, 1,4-pipéridino, –NH– et –N(R°)–, R° ayant l'une des significations de R' ou étant un alcanoyle en $C_2$ à $C_5$, R* est un atome d'hydrogène ou bien un alkyle en $C_1$–$C_4$ ou un aryle éventuellement substitués, Y' le groupe vinyle ou un groupe éthyle avec à la position β un substituant éliminable par un alcali, ou le groupe β-hydroxyéthyle, G représente un liaison directe, un alkylène en $C_1$–$C_8$ à chaîne linéaire ou ramifiée, ou bien un radical bivalent aliphatique-cycloaliphatique ou cycloaliphatique, les radicaux cycloaliphatique ayant chacun de 5 à 8 atomes de carbone, et M est un atome d'hydrogène, un métal alcalin ou l'équivalent d'un métal alcalino-terreux.

21. Un composé de formule générale (3)

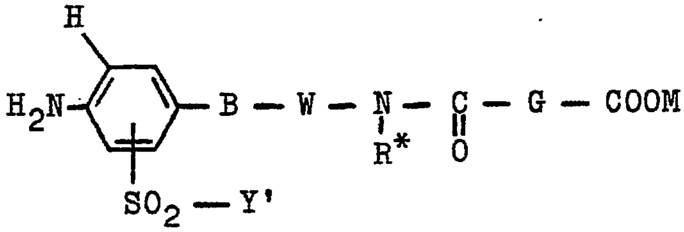

(3)

dans laquelle B est un atome d'oxygène ou de soufre ou un groupe amino –NH– ou –N(R′)–, R′ étant un alkyle en $C_1$–$C_6$, W est un radical bivalent, aliphatique, ou cycloaliphatique en $C_5$–$C_{10}$ avec éventuellement comme substituant un alkyle en $C_1$–$C_4$, ou encore aliphatique-($C_5$ en $C_8$)-cycloaliphatique avec éventuellement comme substituant également un alkyle en $C_1$–$C_4$, les radicaux aliphatiques pouvant être interrompus par des hétérogroupes tels que –O–, –S–, $-SO_2-$, –CO–, 1,4-pipéridino, –NH– et –N(R°)–, R° ayant l'une des significations de R′ ou étant un alcanoyle en $C_2$ à $C_5$, R* est un atome d'hydrogène ou bien un alkyle en $C_1$–$C_4$ ou un aryle éventuellement substitués, Y′ le groupe vinyle, ou un groupe éthyle avec à la position β un substituant éliminable par un alcali, ou le groupe β-hydroxyéthlye, G représente une liaison directe, un alkylène en $C_1$–$C_8$ à chaîne linéaire ou ramifiée, ou bien un radical bivalent aliphatique-cycloaliphatique ou cycloaliphatique, les radicaux cycloaliphatiques ayant chacun de 5 à 8 atomes de carbone, et M est un atome d'hydrogène, un métal alcalin ou l'équivalent d'un métal alcalino-terreux.

22. Composé selon la revendication 19, 20 ou 21, caractérisé en ce que B est le groupe –NH–.

23. Procédé de préparation des composés de formule (9), (8) ou (3) qui ont été définis aux revendications respectives 19, 20 et 21, procédé caractérisé en ce que l'on fait réagir un composé de formule (5)

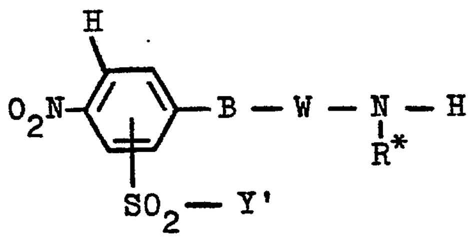

(5)

dans laquelle les divers symboles ont les significations qui ont été données à la revendication 19, 20 ou 21, avec un anhydride de formule (6) ou (7)

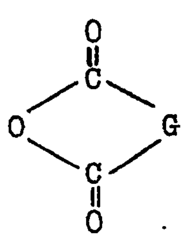

(6)

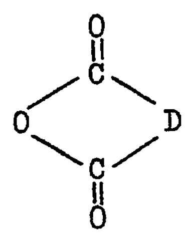

(7)

et le cas échéant on réduit le groupe nitro du composé ainsi formé et on sature une liaison oléfinique éventuellement présente.

24. Emploi d'un composé de formule (9) selon la revendication 19, ou bien d'un composé de formule (8) selon la revendication 20 ou d'un composé de formule (3) selon la revendication 21, pour la synthèse de colorants, en particulier de composés triphénodioxaziniques de formule générale (1) selon la revendication 1.